(19) Europäisches Patentamt
European Patent Office
Office européen des brevets

(11) EP 1 112 266 B1

(12) EUROPÄISCHE PATENTSCHRIFT

(45) Veröffentlichungstag und Bekanntmachung des Hinweises auf die Patenterteilung:
**14.05.2003 Patentblatt 2003/20**

(21) Anmeldenummer: **99944330.2**

(22) Anmeldetag: **04.08.1999**

(51) Int Cl.[7]: **C07D 401/12**, C07D 401/04, C07D 403/04, C07D 417/04, C07D 403/12, A61K 31/505, A61K 31/506, A61P 9/10, A61P 13/12, A61P 11/00

(86) Internationale Anmeldenummer:
**PCT/EP99/05636**

(87) Internationale Veröffentlichungsnummer:
**WO 00/009496 (24.02.2000 Gazette 2000/08)**

(54) **SUBSTITUIERTE 4-AMINO-2-ARYL-PYRIMIDINE, IHRE HERSTELLUNG, IHRE VERWENDUNG UND SIE ENTHALTENDE PHARMAZEUTISCHE PRÄPARATE**

SUBSTITUTED 4-AMINO-2-ARYL-PYRIMIDINES, THEIR PRODUCTION AND USE AND PHARMACEUTICAL PREPARATIONS CONTAINING SAME

4-AMINO-2-ARYL-PYRIMIDINE SUBSTITUEE PROCEDE DE FABRICATION, UTILISATION ET PREPARATIONS PHARMACEUTIQUES LA CONTENANT

(84) Benannte Vertragsstaaten:
**AT BE CH CY DE DK ES FI FR GB GR IE IT LI LU MC NL PT SE**

(30) Priorität: **13.08.1998 DE 19836697**

(43) Veröffentlichungstag der Anmeldung:
**04.07.2001 Patentblatt 2001/27**

(73) Patentinhaber: **Aventis Pharma Deutschland GmbH**
**65929 Frankfurt am Main (DE)**

(72) Erfinder:
- **SCHINDLER, Ursula**
**D-65812 Bad Soden (DE)**
- **SCHÖNAFINGER, Karl**
**D-63755 Alzenau (DE)**
- **STROBEL, Hartmut**
**D-65835 Liederbach (DE)**

(56) Entgegenhaltungen:
**EP-A- 0 055 693**
**EP-A- 0 555 478**
**EP-A- 0 908 456**
**WO-A-98/37079**
**DE-A- 4 034 762**
**DE-A- 19 744 027**
**DE-A- 19 756 388**

- **PATENT ABSTRACTS OF JAPAN vol. 18, no. 542 (C-1261), 17. Oktober 1994 (1994-10-17) & JP 06 192252 A (NISSAN CHEM IND LTD), 12. Juli 1994 (1994-07-12)**
- **NIKOLAENKOVA E B ET AL: "Synthesis of 4,6-bis(1H,1,2,3-triazolyl)pyrimidines by the reaction of 4,6-diazido-2-(methoxyphenyl)pyrimidine with compounds containing a reactive methylene group" CHEM. HETEROCYCL. COMPD. (N. Y.) (CHCCAL,00093122);1998; VOL.33 (8); PP.968-972, XP002123827 Russian Academy of Sciences;Novosibirsk Institute of Organic Chemistry, Siberian Branch; novosibirsk; 630090; Russia (RU)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Verbindungen der Formel 1,

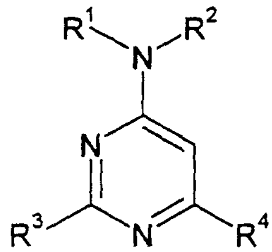

in der $R^1$, $R^2$, $R^3$ und $R^4$ die unten angegebenen Bedeutungen haben, die wertvolle Arzneimittelwirkstoffe für die Therapie und Prophylaxe von Krankheiten sind, zum Beispiel von Herz-Kreislauferkrankungen wie Bluthochdruck, Angina pectoris, Herzinsuffizienz, Thrombosen oder Atherosklerose. Die Verbindungen der Formel I haben die Fähigkeit zur Modulation der körpereigenen Produktion von cyclischem Guanosinmonophosphat (cGMP) und eignen sich generell zur Therapie und Prophylaxe von Krankheitszuständen, die mit einem gestörten cGMP-Haushalt verbunden sind. Die Erfindung betrifft weiterhin Verfahren zur Herstellung von Verbindungen der Formel I, ihre Verwendung zur Therapie und Prophylaxe der bezeichneten Krankheitszustände und, zur Herstellung von Arzneimitteln dafür, sowie pharmazeutische Präparate, die Verbindungen der Formel I enthalten.

cGMP ist ein wichtiger intrazellulärer Botenstoff, der über die Modulation cGMPabhängiger Proteinkinasen, Phosphodiesterasen und Ionenkanäle eine Reihe von pharmakologischen Effekten auslöst. Beispiele sind die Glattmuskelrelaxation, die Inhibition der Thrombozytenaktivierung und die Hemmung der Glattmuskelzellproliferation und Leukozytenadhäsion. cGMP wird durch partikuläre und lösliche Guanylatcyclasen als Antwort auf eine Reihe extrazellulärer und intrazellulärer Stimuli produziert. Im Falle der partikulären Guanylatcyclasen erfolgt die Stimulation im wesentlichen durch peptidische Signalstoffe, wie dem atrialen natriuretischen Peptid oder dem cerebralen natriuretischen Peptid. Die löslichen Guanylatcyclasen (sGC), bei denen es sich um cytosolische, heterodimere Hämproteine handelt, werden dagegen im wesentlichen durch eine Familie niedermolekularer, enzymatisch gebildeter Faktoren reguliert. Wichtigstes Stimulans ist das Stickstoffmonoxid (NO) oder eine nahe verwandte Spezies. Die Bedeutung anderer Faktoren wie Kohlenmonoxid oder dem Hydroxylradikal ist noch weitgehend ungeklärt. Als Aktivierungsmechanismus der Aktivierung durch NO wird die Anbindung von NO an das Häm unter Ausbildung eines pentakoordinierten Häm-Nitrosyl-Komplexes diskutiert. Die damit verbundene Freisetzung des im Basal-Zustand an das Eisen gebundenen Histidins überführt das Enzym in die aktivierte Konformation.

Aktive lösliche Guanylatcyclasen setzen sich aus je einer α- und einer β-Untereinheit zusammen. Von den Untereinheiten wurden mehrere Subtypen beschrieben, die sich untereinander bezüglich Sequenz, gewebespezifischer Verteilung und Expression in verschiedenen Entwicklungsstadien unterscheiden. Die Subtypen $\alpha_1$ und $\beta_1$ werden hauptsächlich in Gehirn und Lunge exprimiert, während $\beta_2$ vor allem in Leber und Niere gefunden wird. In humanem fötalen Gehirn konnte der Subtyp $\alpha_2$ nachgewiesen werden. Die als $\alpha_3$ und $\beta_3$ bezeichneten Untereinheiten wurden aus menschlichem Gehirn isoliert und sind homolog zu $\alpha_1$ und $\beta_1$. Neuere Arbeiten weisen auf eine $\alpha_{2i}$-Untereinheit hin, die ein Insert in der katalytischen Domäne enthält. Alle Untereinheiten zeigen große Homologien im Bereich der katalytischen Domäne. Die Enzyme enthalten vermutlich ein Häm pro Heterodimer, das über $\beta_1$-Cys-78 und/oder $\beta_1$-His-105 gebunden ist und Teil des regulatorischen Zentrums ist.

Unter pathologischen Bedingungen kann die Bildung Guanylatcyclase-aktivierender Faktoren vermindert sein oder es kann durch das vermehrte Auftreten freier Radikale ein verstärkter Abbau derselben erfolgen. Die daraus resultierende verminderte Aktivierung der sGC führt über die Abschwächung der jeweiligen cGMPvermittelten Zellantwort beispielsweise zum Anstieg des Blutdrucks, zur Plättchenaktivierung oder zu vermehrter Zellproliferation und Zelladhäsion. Als Folge kommt es zur Ausbildung von endothelialer Dysfunktion, Atherosklerose, Bluthochdruck, stabiler oder instabiler Angina pectoris, Thrombosen, Myocardinfarkt, Schlaganfällen oder erektiler Dysfunktion. Die pharmakologische Stimulation der sGC bietet eine Möglichkeit zur Normalisierung der cGMP-Produktion und erlaubt damit die Behandlung bzw. Prävention derartiger Krankheiten.

Zur pharmakologischen Stimulation der sGC wurden bisher fast ausschließlich Verbindungen verwendet, deren Wirkung auf einer intermediären NO-Freisetzung beruht, beispielsweise organische Nitrate. Der Nachteil dieser Behandlungsweise liegt in der Toleranzentwicklung und Wirkungsabschwächung und der deshalb erforderlich werdenden höheren Dosierung.

Verschiedene nicht über eine NO-Freisetzung wirkende sGC-Stimulatoren wurden von Vesely in einer größeren Zahl von Arbeiten beschrieben. Die Verbindungen, bei denen es sich zumeist um Hormone, Pflanzenhormone, Vitamine oder zum Beispiel Naturstoffe wie Echsengifte handelt, zeigen jedoch durchweg nur schwache Effekte auf die cGMP-Bildung in Zellysaten (D. L. Vesely, Eur. J. Clin. Invest. 15 (1985) 258; D. L. Vesely, Biochem. Biophys. Res. Comm. 88 (1979) 1244). Eine Stimulation Häm-freier Guanylatcyclase durch Protoporphyrin IX wurde durch Ignarro et al. (Adv. Pharmacol. 26 (1994) 35) nachgewiesen. Pettibone et al. (Eur. J. Pharmacol. 116 (1985) 307) beschrieben für Diphenyliodoniumhexafluorophoshat eine blutdrucksenkende Wirkung und führten diese auf eine Stimulation der sGC zurück. Isoliquiritiginin, das an isolierten Rattenaorten eine relaxierende Wirkung zeigt, aktiviert laut Yu et al. (Brit. J. Pharmacol. 114 (1995) 1587) ebenfalls die sGC. Ko et al. (Blood 84 (1994) 4226), Yu et al. (Biochem. J. 306 (1995) 787) und Wu et al. (Brit. J. Pharmacol. 116 (1995) 1973) wiesen eine sGC-stimulierende Aktivität von 1-Benzyl-3-(5-hydroxymethyl-2-furyl)-indazol nach und demonstrierten eine antiproliferative und thrombozytenhemmende Wirkung. Substituierte Pyrazole und kondensierte Pyrazole, die eine sGC-stimulierende Wirkung haben, werden in der EP-A-908456 und der DE-A-19744027 beschrieben, substituierte Chinazoline mit einer derartigen Wirkung in der DE-A-19756388.

Verschiedene 4-Amino-2-aryl-pyrimidine sind bereits bekannt. So werden in der EP-A-55693 Pyrimidine beschrieben, die in der 2-Position durch eine Phenylgruppe substituiert sind und die sich als Gegenmittel zum Schutz von Kulturpflanzen vor der phytotoxischen Wirkung von Herbiziden eignen. In der EP-A-136976 werden 2-Phenylpyrimidine beschrieben, die Regulatoren des Pflanzenwuchses sind. Für bestimmte 2-Phenylpyrimidine, die in der 4-Position unter anderem eine Aminogruppe als Substituenten tragen können, wird in der EP-A-555478 beschrieben, daß sie das Lernvermögen und die Gedächtnisleistung verbessern.

Überraschend wurde nun gefunden, daß die erfindungsgemäßen Pyrimidine der Formel I eine starke Guanylatcyclase-Aktivierung bewirken, aufgrund derer sie zur Therapie und Prophylaxe von Krankheiten geeignet sind, die mit einem niedrigen cGMP-Spiegel verbunden sind.

Die vorliegende Erfindung betrifft somit Verbindungen der Formel I,

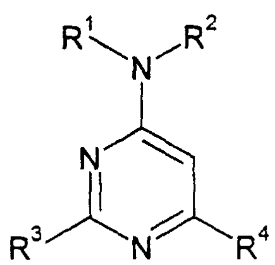

in der

$R^1$ für ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, $R^5R^6N$ und Aryl substituiert sein kann, ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, oder den Rest eines 5-gliedrigen bis 7-gliedrigen gesättigten heterocyclischen Ringes, der ein oder zwei gleiche oder verschiedene Hetero-Ringglieder aus der Reihe O, $NR^7$ und $S(O)_m$ enthält und der durch einen oder mehrere gleiche oder verschiedene Substituenten aus der Reihe ($C_1$-$C_4$)-Alkyl und Aryl-($C_1$-$C_4$)-alkyl- substituiert sein kann, steht;

und

$R^2$ für Wasserstoff, ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, $R^5R^6N$ und Aryl substituiert sein kann, ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, oder den Rest eines 5-gliedrigen bis 7-gliedrigen gesättigten heterocyclischen Ringes, der ein oder zwei gleiche oder verschiedene Hetero-Ringglieder aus der Reihe O, $NR^7$ und $S(O)_m$ enthält und der durch einen oder mehrere gleiche oder verschiedene Substituenten aus der Reihe ($C_1$-$C_4$)-Alkyl und Aryl-($C_1$-$C_4$)-alkyl- substituiert sein kann, steht;

oder

$R^1R^2N$ für einen über ein Ringstickstoffatom gebundenen Rest eines 5-gliedrigen bis 7-gliedrigen gesättigten heterocyclischen Ringes steht, der neben dem die Reste $R^1$ und $R^2$ tragenden Stickstoffatom ein weiteres Hetero-Ringglied aus der Reihe O, $NR^7$ und $S(O)_m$ enthalten kann und der durch einen oder mehrere gleiche oder verschiedene Substituenten aus der Reihe ($C_1$-$C_4$)-Alkyl, Hydroxy, ($C_1$-$C_4$)-Alkoxy, $R^8R^9N$, Hydroxycarbonyl, ($C_1$-$C_4$)-Alkoxycarbonyl

und $R^8R^9N$-CO- substituiert sein kann;
$R^3$ für Phenyl steht, das durch einen oder mehrere gleiche oder verschiedene Substituenten aus der Reihe Halogen, ($C_1$-$C_4$)-Alkyl, Phenyl, $CF_3$, $NO_2$, OH, -O-($C_1$-$C_4$)-Alkyl, -O-($C_2$-$C_4$)-Alkyl-O-($C_1$-$C_4$)-Alkyl, ($C_1$-$C_2$)-Alkylendioxy, $NH_2$, -NH-($C_1$-$C_4$)-Alkyl, -N(($C_1$-$C_4$)-Alkyl)$_2$, -NH-CHO, -NH-CO-($C_1$-$C_4$)-Alkyl, -CN, -CO-$NH_2$, -CO-NH-($C_1$-$C_4$)-Alkyl, -CO-N(($C_1$-$C_4$)-Alkyl)$_2$, -CO-OH, -CO-O-($C_1$-$C_4$)-Alkyl, -CHO und -CO-($C_1$-$C_4$)-Alkyl substituiert sein kann;
$R^4$ für ($C_2$-$C_5$)-Alkyl, Trifluormethyl oder Phenyl, das durch einen oder mehrere gleiche oder verschiedene Substituenten aus der Reihe Halogen, ($C_1$-$C_4$)-Alkyl, Phenyl, $CF_3$, $NO_2$, OH, -O-($C_1$-$C_4$)-Alkyl, -O-($C_2$-$C_4$)-Alkyl-O-($C_1$-$C_4$)-Alkyl, ($C_1$-$C_2$)-Alkylendioxy, $NH_2$, -NH-($C_1$-$C_4$)-Alkyl, -N(($C_1$-$C_4$)-Alkyl)$_2$, -NH-CHO, -NH-CO-($C_1$-$C_4$)-Alkyl, -CN, -CO-$NH_2$, -CO-NH-($C_1$-$C_4$)-Alkyl, -CO-N(($C_1$-$C_4$)-Alkyl)$_2$, -CO-OH, -CO-O-($C_1$-$C_4$)-Alkyl, -CHO und -CO-($C_1$-$C_4$)-Alkyl substituiert sein kann, steht;
$R^5$ und $R^6$ für gleiche oder verschiedene Reste aus der Reihe Wasserstoff und ($C_1$-$C_4$)-Alkyl stehen oder die Gruppe $R^5R^6N$ für einen über ein Ringstickstoffatom gebundenen Rest eines 5-gliedrigen bis 7-gliedrigen gesättigten oder ungesättigten heterocyclischen Ringes steht, der neben dem die Reste $R^5$ und $R^6$ tragenden Stickstoffatom als weiteres Hetero-Ringglied noch ein Sauerstoffatom, eine Gruppe $S(O)_m$ oder ein Stickstoffatom enthalten kann und der an Ringkohlenstoffatomen einen oder mehrere gleiche oder verschiedene Substituenten aus der Reihe ($C_1$-$C_4$)-Alkyl, Hydroxy und Amino tragen kann und an einem Ringstickstoffatom einen Rest $R^7$ tragen kann;
$R^7$ für Wasserstoff, ($C_1$-$C_4$)-Alkyl, Aryl-($C_1$-$C_4$)-alkyl-, Hydroxy-($C_1$-$C_4$)-alkyl-, Hydroxycarbonyl-($C_1$-$C_4$)-alkyl-, (($C_1$-$C_4$)-Alkoxycarbonyl)-($C_1$-$C_4$)-alkyl-, $R^8R^9N$-CO-($C_1$-$C_4$)-alkyl-, $R^{10}$-$SO_2$- oder Aryl steht, wobei $R^7$ dann, wenn diese Gruppe an einem für $R^1R^2N$ stehenden Piperazinorest steht, nicht für carbocyclisches Aryl oder carbocyclisches Aryl-($C_1$-$C_4$)-alkyl- stehen kann;
$R^8$ und $R^9$ für gleiche oder verschiedene Reste aus der Reihe Wasserstoff und ($C_1$-$C_4$)-Alkyl stehen;
$R^{10}$ für ($C_1$-$C_4$)-Alkyl, Aryl oder $R^8R^9N$ steht;
Aryl für Phenyl, Naphthyl oder Heteroaryl steht, die alle durch einen oder mehrere gleiche oder verschiedene Substituenten aus der Reihe Halogen, ($C_1$-$C_4$)-Alkyl, Phenyl, $CF_3$, $NO_2$, OH, -O-($C_1$-$C_4$)-Alkyl, -O-($C_2$-$C_4$)-Alkyl-O-($C_1$-$C_4$)-Alkyl, ($C_1$-$C_2$)-Alkylendioxy, $NH_2$, -NH-($C_1$-$C_4$)-Alkyl, -N(($C_1$-$C_4$)-Alkyl)$_2$, -NH-CHO, -NH-CO-($C_1$-$C_4$)-Alkyl, -CN, -CO-$NH_2$, -CO-NH-($C_1$-$C_4$)-Alkyl, -CO-N(($C_1$-$C_4$)-Alkyl)$_2$, -CO-OH, -CO-O-($C_1$-$C_4$)-Alkyl, -CHO und -CO-($C_1$-$C_4$)-Alkyl substituiert sein können;
Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere gleiche oder verschiedene Ringheteroatome aus der Reihe N, O und S enthalten;
m für 0, 1 oder 2 steht;
in allen ihren stereoisomeren Formen und Mischungen davon in allen Verhältnissen, und ihre physiologisch verträglichen Salze,
wobei Verbindungen der Formel I ausgeschlossen sind, in denen gleichzeitig $R^4$ für tert-Butyl oder Trifluormethyl steht, $R^3$ für Phenyl steht, das durch einen oder zwei gleiche oder verschiedene Substituenten aus der Reihe Halogen, OH, -O-$R^{11}$ und $CF_3$ substituiert sein kann, $R^1R^2N$ für $R^{11}$-NH-, $(R^{11})_2N$- oder $R^{12}R^{13}N$-$(CH_2)_p$-NHsteht, p für 2 oder 3 steht, $R^{11}$ für gesättigtes unsubstituiertes ($C_1$-$C_4$)-Alkyl steht und $R^{12}$ und $R^{13}$ für gleiche oder verschiedene Reste aus der Reihe Wasserstoff und $R^{11}$ stehen oder die Gruppe $R^{12}R^{13}N$ für einen über ein Ringstickstoffatom gebundenen Rest eines 5-gliedrigen oder 6-gliedrigen gesättigten heterocyclischen Ringes steht, der neben dem die Reste $R^{12}$ und $R^{13}$ tragenden Stickstoffatom als weiteres Hetero-Ringglied noch ein Sauerstoffatom, ein Schwefelatom oder ein Stickstoffatom enthalten kann und der durch einen Arylrest oder durch einen Aryl-($C_1$-$C_4$)-alkylrest substituiert sein kann, wobei die Arylgruppe durch einen oder zwei gleiche oder verschiedene Substituenten aus der Reihe Halogen, OH, -O-$R^{11}$ und $CF_3$ substituiert sein kann.

Können Gruppen oder Substituenten mehrfach in den Verbindungen der Formel I vorkommen, so können sie alle unabhängig voneinander die angegebenen Bedeutungen haben und können jeweils gleich oder verschieden sein.

Alkylreste können geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind. Beispiele für Alkylgruppen sind Methyl, Ethyl, Propyl, Butyl, Pentyl, Hexyl, Heptyl, Octyl, die n-Isomeren dieser Reste, Isopropyl, Isobutyl, Isopentyl, sec-Butyl, tert-Butyl, Neopentyl, 3,3-Dimethylbutyl. Unter dem Begriff Alkyl sind hier neben gesättigten Alkylresten ausdrücklich auch ungesättigte Alkylreste zu verstehen, also Alkylreste, die eine oder mehrere Doppelbindungen und/oder eine oder mehrere Dreifachbindungen enthalten, zum Beispiel Alkenylreste und Alkinylreste. Naturgemäß muß ein ungesättigter Alkylrest mindestens zwei Kohlenstoffatome enthalten, eine ($C_1$-$C_8$)-Alkylgruppe zum Beispiel umfaßt also gesättigte ($C_1$-$C_8$)-Alkylreste und ungesättigte ($C_2$-$C_8$)-Alkylreste, ein ($C_1$-$C_4$)-Alkylrest umfaßt gesättigte ($C_1$-$C_4$)-Alkylreste und ungesättigte ($C_2$-$C_4$)-Alkylreste. Beispiele für ungesättigte Alkylreste sind der Vinylrest, der 2-Propenylrest (Allylrest), der 2-Butenylrest, der 2-Methyl-2-propenylrest, der Ethinylrest, der 2-Propinylrest (Propargylrest) oder der 3-Butinylrest. Sind Alkylreste durch einen oder mehrere Substituenten substituiert, so sind sie bevorzugt durch einen, zwei oder drei, insbesondere durch einen oder zwei gleiche oder verschiedene Substituenten substituiert. Substituenten können sich an beliebigen Kohlenstoffatomen des Alkylrestes befinden.

Cycloalkyl ist beispielsweise Cyclopropyl, Cyclobutyl, Cyclopentyl, Cyclohexyl, Cycloheptyl, Cyclooctyl oder Cyclononyl, die alle auch wie angegeben substituiert sein können, zum Beispiel durch einen oder mehrere gleiche oder verschiedene ($C_1$-$C_4$)-Alkylreste, insbesondere durch Methyl, und/oder durch Hydroxy. Sind Cycloalkylreste durch einen oder mehrere Substituenten substituiert, so sind sie bevorzugt durch einen, zwei, drei oder vier, insbesondere durch einen oder zwei, gleiche oder verschiedene Substituenten substituiert. Beispiele für solche substituierten Cycloalkylreste sind 4-Methylcyclohexyl, 4-tert-Butylcyclohexyl, 4-Hydroxycyclohexyl, 4-Aminocyclohexyl oder 2,3-Dimethylcyclopentyl. Substituenten können sich an beliebigen Kohlenstoffatomen des Cycloalkylrestes befinden.

Carbocyclische Arylreste wie Phenylreste und Naphthylreste und Heteroarylreste, können, soweit nicht anders angegeben, unsubstituiert sein oder einen oder mehrere, zum Beispiel ein, zwei, drei oder vier, gleiche oder verschiedene Substituenten tragen, die sich in beliebigen Positionen befinden können. Soweit nicht anders angegeben, können in diesen Resten als Substituenten zum Beispiel die in der Definition der Gruppe Aryl angegebenen Substituenten auftreten. Sind in Verbindungen der Formel I Nitrogruppen als Substituenten vorhanden, so können insgesamt nur bis zu zwei Nitrogruppen im Molekül vorhanden sein. Trägt ein Arylrest wie zum Beispiel ein Phenylrest wiederum einen Phenylrest als Substituenten, so kann in letzterem der Benzolring auch wiederum unsubstituiert sein oder durch einen oder mehrere, zum Beispiel ein, zwei, drei oder vier, gleiche oder verschiedene Reste substituiert sein, zum Beispiel durch Reste aus der Reihe ($C_1$-$C_4$)-Alkyl, Halogen, Hydroxy, ($C_1$-$C_4$)-Alkoxy, Trifluormethyl, Cyan, Hydroxycarbonyl, (($C_1$-$C_4$)-Alkoxy)carbonyl, Aminocarbonyl, Nitro, Amino, ($C_1$-$C_4$)-Alkylamino, Di-(($C_1$-$C_4$)-alkyl)amino und (($C_1$-$C_4$)-Alkyl)carbonylamino.

In monosubstituierten Phenylresten kann sich der Substituent in der 2-Position, der 3-Position oder der 4-Position befinden, in disubstituierten Phenylresten können sich die Substituenten in 2,3-Position, 2,4-Position, 2,5-Position, 2,6-Position, 3,4-Position oder 3,5-Position befinden. In trisubstituierten Phenylresten können sich die Substituenten in 2,3,4-Position, 2,3,5-Position, 2,3,6-Position, 2,4,5-Position, 2,4,6-Position oder 3,4,5-Position befinden. Naphthyl kann 1-Naphthyl oder 2-Naphthyl sein. In monosubstituierten 1-Naphthylresten kann sich der Substituent in der 2-Position, der 3-Position, der 4-Position, der 5-Position, der 6-Position, der 7-Position oder der 8-Position befinden, in monosubstituierten 2-Naphthylresten in der 1-Position, der 3-Position, der 4-Position, der 5-Position, der 6-Position, der 7-Position oder der 8-Position. Auch in mehrfach substituierten Naphthylresten, zum Beispiel zweifach oder dreifach substituierten Naphthylresten, können sich die Substituenten in allen möglichen Positionen befinden.

Soweit nicht anders angegeben, leiten sich Heteroarylreste, Reste von gesättigten heterocyclischen Ringen und Reste von Ringen, die von zwei an ein Stickstoffatom gebundenen Gruppen zusammen mit diesem Stickstoffatom gebildet werden, bevorzugt von Heterocyclen ab, die ein, zwei, drei oder vier gleiche oder verschiedene Ringheteroatome enthalten, besonders bevorzugt von Heterocyclen, die ein oder zwei oder drei, insbesondere ein oder zwei, gleiche oder verschiedene Ringheteroatome enthalten. Soweit nicht anders angegeben, können die Heterocyclen monocyclisch oder polycyclisch sein, zum Beispiel monocyclisch, bicyclisch oder tricyclisch. Bevorzugt sind sie monocyclisch oder bicyclisch, insbesondere monocyclisch. Die einzelnen Ringe enthalten bevorzugt 5, 6 oder 7 Ringglieder. Beispiele für monocyclische und bicyclische heterocyclische Systeme, von denen sich in den Verbindungen der Formel I vorkommende Reste ableiten können, sind Pyrrol, Furan, Thiophen, Imidazol, Pyrazol, 1,2,3-Triazol, 1,2,4-Triazol, 1,3-Dioxol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin, 1,3-Thiazepin, Indol, Benzothiophen, Benzofuran, Benzothiazol, Benzimidazol, Chinolin, Isochinolin, Cinnolin, Chinazolin, Chinoxalin, Phthalazin, Thienothiophene, 1,8-Naphthyridin und andere Naphthyridine, Pteridin, oder Phenothiazin, alle jeweils in gesättigter Form (Perhydro-Form) oder in teilweise ungesättigter Form (zum Beispiel Dihydro-Form und Tetrahydro-Form) oder in maximal ungesättigter Form, soweit die betreffenden Formen bekannt und stabil sind. Zu den in Betracht kommenden Heterocyclen gehören somit beispielsweise auch die gesättigten Heterocyclen Pyrrolidin, Piperidin, Perhydroazepin (Hexamethylenimin), Piperazin, Morpholin, 1,3-Thiazolidin und Thiomorpholin. Der Sättigungsgrad von heterocyclischen Gruppen ist bei den einzelnen Definitionen angegeben. Ungesättigte Heterocyclen können zum Beispiel eine, zwei oder drei Doppelbindungen im Ringsystem enthalten. 5-Ringe und 6-Ringe in monocyclischen und polycyclischen Heterocyclen können insbesondere auch aromatisch sein.

Heterocyclische Reste können über jedes geeignete Ringkohlenstoffatom gebunden sein. Stickstoffheterocyclen, zum Beispiel Pyrrol, Imidazol, Pyrrolidin, Piperidin, Hexamethylenimin, 1,3-Thiazolidin, Morpholin, Thiomorpholin, Piperazin etc., können auch über jedes geeignete Ringstickstoffatom gebunden sein, insbesondere, wenn der betreffende Stickstoffheterocyclus an ein Kohlenstoffatom gebunden ist.

Beispielsweise kann ein Thienylrest als 2-Thienylrest oder 3-Thienylrest vorliegen, ein Furanrest als 2-Furylrest oder 3-Furylrest, ein Pyridylrest als 2-Pyridylrest, 3-Pyridylrest oder 4-Pyridylrest, ein Piperidinrest als 1-Piperidinylrest (= Piperidinorest), 2-Piperidinylrest, 3-Piperidinylrest oder 4-Piperidinylrest, ein (Thio)Morpholinrest als 2-(Thio) Morpholinylrest, 3-(Thio)Morpholinylrest oder 4-(Thio)Morpholinylrest (= (Thio)Morpholinorest). Ein Rest, der sich vom 1,3-Thiazol ableitet, kann über die 2-Position, die 3-Position, die 4-Position oder die 5-Position gebunden sein, ein Rest, der sich vom Imidazol ableitet, über die 1-Position, die 2-Position, die 4-Position oder die 5-Position.

Soweit nicht anders angegeben, können die heterocyclischen Gruppen unsubstituiert sein oder einen oder mehrere, zum Beispiel einen, zwei, drei oder vier, gleiche oder verschiedene Substituenten tragen. Die Substituenten in Heterocyclen können sich in beliebigen Positionen befinden, beispielsweise in einem 2-Thienylrest oder 2-Furylrest in der 3-Position und/oder in der 4-Position und/oder in der 5-Position, in einem 3-Thienylrest oder 3-Furylrest in der 2-Position und/oder in der 4-Position und/oder in der 5-Position, in einem 2-Pyridylrest in der 3-Position und/oder in der 4-Position und/oder in der 5-Position und/oder in der 6-Position, in einem 3-Pyridylrest in der 2-Position und/oder in der 4-Position und/oder in der 5-Position und/oder in der 6-Position, in einem 4-Pyridylrest in der 2-Position und/oder in der 3-Position und/oder in der 5-Position und/oder in der 6-Position. Soweit nicht anders angegeben, können als Substituenten zum Beispiel die in der Definition der Gruppe Aryl angegebenen Substituenten auftreten, im Falle von gesättigten oder teilweise ungesättigten Heterocyclen als weiterere Substituenten auch die Oxogruppe und die Thioxogruppe. Substituenten an einem Heterocyclus wie auch Substituenten an einem Carbocyclus können auch einen Ring bilden, es können also an ein Ringsystem weitere Ringe ankondensiert sein, so daß zum Beispiel cyclopenta-kondensierte, cyclohexa-kondensierte oder benzo-kondensierte Ringe vorliegen können. Soweit nicht anders angegeben, kommen als Substituenten an einem substituierbaren Stickstoffatom eines Heterocyclus zum Beispiel unsubstituierte und substituierte ($C_1$-$C_4$)-Alkylreste, Arylreste, Acylreste wie -CO-($C_1$-$C_4$)-Alkyl oder -CO-Aryl, oder Sulfonylreste wie -$SO_2$-($C_1$-$C_4$)-Alkyl oder -$SO_2$-Aryl in Betracht. Geeignete Schwefelheterocyclen können auch als S-Oxide oder S,S-Dioxide vorliegen, das heißt, sie können an Stelle eines Schwefelatoms die Gruppe S(=O) oder die Gruppe $S(=O)_2$ enthalten. Geeignete Stickstoffheterocyclen können auch als N-Oxide oder als Quartärsalze mit einem von einer physiologisch verträglichen Säure abgeleiteten Anion als Gegenion vorliegen. Pyridylreste können zum Beispiel als Pyridin-N-oxide vorliegen.

Halogen bedeutet Fluor, Chlor, Brom oder Iod, vorzugsweise Fluor oder Chlor.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie, zum Beispiel an Doppelbindungen oder Cycloalkylgruppen, sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel 1, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine (Zwitterionen) zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen.

Die vorliegende Erfindung umfaßt weiterhin alle Solvate von Verbindungen der Formel I, zum Beispiel Hydrate

oder Addukte mit Alkoholen, sowie Derivate der Verbindungen der Formel I wie zum Beispiel Ester und Amide, und Pro-Drugs und aktive Metabolite.

Bevorzugt steht $R^1$ für ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, $R^5R^6N$ und Aryl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann. Bevorzugt steht $R^2$ für Wasserstoff, ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-$S(O)_m$-, $R^5R^6N$ und Aryl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann. Besonders bevorzugt ist es, wenn $R^1$ für ($C_1$-$C_8$)-Alkyl oder ($C_3$-$C_9$)-Cycloalkyl steht und $R^2$ für Wasserstoff steht oder wenn $R^1$ und $R^2$ für gleiches oder verschiedenes ($C_1$-$C_8$)-Alkyl stehen, wobei alle Reste unsubstituiert oder wie angegeben substituiert sein können. Ganz besonders bevorzugt ist, wenn $R^1$ für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, steht, und $R^2$ für Wasserstoff steht. Wenn $R^1$ für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, oder für den Rest eines 5-gliedrigen, 6-gliedrigen oder 7-gliedrigen gesättigten heterocyclischen Ringes, der ein oder zwei gleiche oder verschiedene Hetero-Ringglieder aus der Reihe O, $NR^7$ und $S(O)_m$ enthält und der durch einen oder mehrere gleiche oder verschiedene Substituenten aus der Reihe ($C_1$-$C_4$)-Alkyl und Aryl-($C_1$-$C_4$)-alkylsubstituiert sein kann, steht, dann steht $R^2$ bevorzugt für Wasserstoff. Ein für $R^1$ oder $R^2$ stehender Alkylrest ist bevorzugt ein unsubstituierter oder substituierter ($C_1$-$C_4$)-Alkylrest. Ein für $R^1$ oder $R^2$ stehender ($C_3$-$C_9$)-Cycloalkylrest ist bevorzugt ein unsubstituierter oder substituierter ($C_3$-$C_7$)-Cycloalkylrest.

Neben den vorstehend genannten bevorzugten Bedeutungen von $R^1$ und $R^2$ ist es weiterhin bevorzugt, wenn die Gruppe $R^1R^2N$ für einen über ein Ringstickstoffatom gebundenen Rest eines 5-gliedrigen, 6-gliedrigen oder 7-gliedrigen gesättigten heterocyclischen Ringes steht, der neben dem die Reste $R^1$ und $R^2$ tragenden Stickstoffatom als weiteres Hetero-Ringglied noch ein Sauerstoffatom, eine Gruppe $S(O)_m$ oder ein einen Rest $R^7$ tragendes Stickstoffatom enthalten kann und der durch einen oder mehrere gleiche oder verschiedene Substituenten aus der Reihe ($C_1$-$C_4$)-Alkyl, Hydroxy, ($C_1$-$C_4$)-Alkoxy, $R^8R^9N$, Hydroxycarbonyl, ($C_1$-$C_4$)-Alkoxycarbonyl und $R^8R^9N$-CO- substituiert sein kann. Ein für $R^1R^2N$ stehender Rest eines heterocyclischen Ringes leitet sich bevorzugt von einem 5-gliedrigen oder 6-gliedrigen gesättigen heterocyclischen Ring ab, besonders bevorzugt vom Piperidin, Morpholin, Thiomorpholin (und seinem S-Oxid und S,S-Dioxid) oder Piperazin, die alle wie angegeben substituiert sein können, ganz besonders bevorzugt vom unsubstituierten Piperidin, Morpholin oder Thiomorpholin (und seinem S-Oxid und S,S-Dioxid) oder vom N-Methylpiperazin.

Die für $R^3$ stehende Phenylgruppe ist bevorzugt eine substituierte Phenylgruppe, besonders bevorzugt eine Phenylgruppe, die durch einen oder zwei der oben in der Definition der Gruppe $R^3$ angegebenen Substituenten substituiert ist. Speziell bevorzugt steht $R^3$ für Phenyl, das durch ein oder zwei gleiche oder verschiedene Substituenten aus der Reihe Halogen und ($C_1$-$C_4$)-Alkyl substituiert ist, darüber hinaus bevorzugt für Phenyl, das durch Chlor oder Methyl substituiert ist. Der Substituent in einer für $R^3$ stehenden einfach substituierten Phenylgruppe steht bevorzugt in der para-Position.

$R^4$ steht bevorzugt für geradkettiges oder verzweigtes ($C_3$-$C_4$)-Alkyl, zum Beispiel n-Propyl, Isopropyl, n-Butyl, Isobutyl oder tert-Butyl.

Aryl steht bevorzugt für Phenyl oder 5-gliedriges oder 6-gliedriges monocyclisches Heteroaryl mit einem oder zwei, insbesondere einem Heteroatom aus der Reihe N, O und S, die wie angegeben substituiert sein können, besonders bevorzugt für unsubstituiertes oder substituiertes Phenyl oder unsubstituiertes Pyridyl, Thienyl oder Furyl, ganz besonders bevorzugt für unsubstituiertes oder substituiertes Phenyl oder unsubstituiertes Pyridyl.

Bevorzugte Verbindungen der Formel I sind solche, in denen einer oder mehrere der darin enthaltenen Reste bevorzugte Bedeutungen haben, wobei alle Kombinationen von bevorzugten Substituentendefinitionen Gegenstand der vorliegenden Erfindung sind. Auch von allen bevorzugten Verbindungen der Formel I umfaßt die vorliegende Erfindung alle ihre stereoisomeren Formen und Mischungen davon in allen Verhältnissen, und ihre physiologisch verträglichen Salze.

Gegenstand der vorliegenden Erfindung sind auch Verfahren zur Herstellung der Verbindungen der Formel I, die im folgenden erläutert sind und nach denen die erfindungsgemäßen Verbindungen erhältlich sind. Die Herstellung der Verbindungen der Formel I kann erfolgen, indem zunächst in an sich bekannter Weise ein Amidin der Formel II mit einem in der 3-Position einen Rest $R^4$ tragenden

3-Oxopropionsäureeester der Formel III zu einem 4-Hydroxypyrimidin der Formel IV umgesetzt wird. R in der Formel III steht zum Beispiel für ($C_1$-$C_4$)-Alkyl wie Methyl oder Ethyl. Das Hydroxypyrimidin der Formel IV wird anschließend aktiviert, zum Beispiel durch Überführung in ein 4-Halogenpyrimidin. Beispielsweise läßt sich die Verbindung der Formel IV durch Umsetzung mit einem Phosphorhalogenid wie Phosphoroxychlorid in das 4-Chlorpyrimidin der Formel V überführen. Durch Umsetzung der Verbindung der Formel V (oder eines anderen reaktiven Derivats des Hydroxypyrimidins) mit dem gewünschten Amin der Formel VI wird dann unter Austausch des Chlors gegen die Aminogruppe die erfindungsgemäße Verbindung der Formel I erhalten. Geeignete Lösungsmittel für diese Austauschreaktion sind zum Beispiel Wasser, Alkohole wie Methanol, Ethanol oder Isopropanol, Ether wie Tetrahydrofuran oder Dioxan, Amide wie Dimethylformamid (DMF) oder N-Methylpyrrolidon (NMP), oder Kohlenwasserstoffe oder halogenierte Kohlenwasserstoffe wie Benzol, Toluol, Xylol, Chlorbenzol oder Dichlorbenzol.

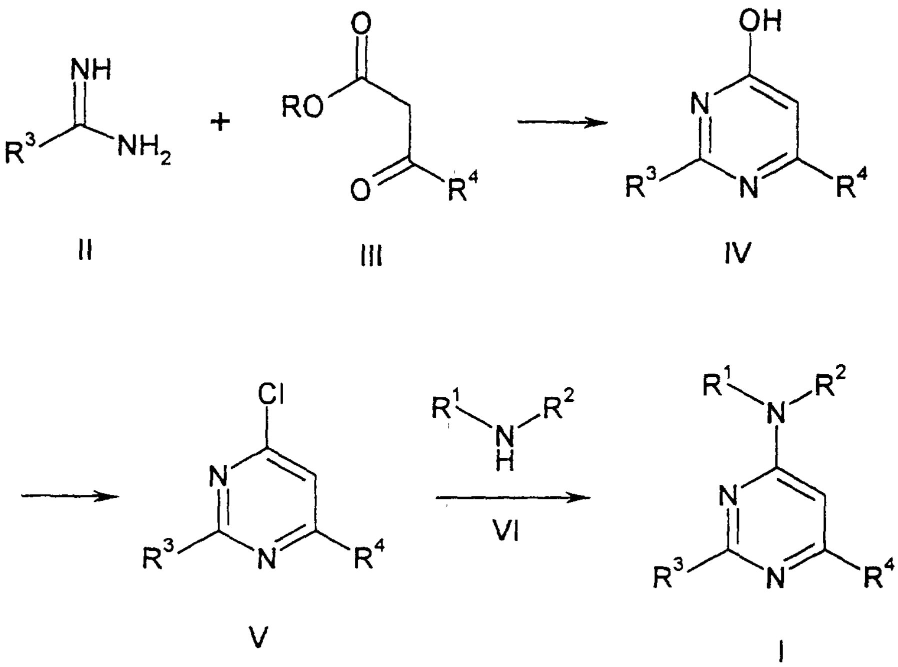

II III IV

V VI I

Die Umsetzungen zur Synthese der Verbindungen der Formel I können in einem weiten Temperaturbereich durchgeführt werden. Bevorzugt sind Reaktionstemperaturen von 20°C bis 150°C. Die Umsetzungen können durch Zusatz von geeigneten Basen wie zum Beispiel Natriumbicarbonat, Natriumcarbonat, Kaliumcarbonat, Natriumalkoholaten, Triethylamin oder Pyridin beschleunigt werden, im ersten und im letzten Schritt zusätzlich auch durch einen Überschuß an Amidin der Formel II bzw. Amin der Formel VI. An Stelle der freien Amidine der Formel II können auch die entsprechenden Amidiniumsalze eingesetzt werden. In diesem Falle ist es besonders günstig, den ersten Schritt unter Zusatz von Basen durchzuführen. Die Zwischenstufen der Formel IV und V und die Endverbindungen der Formel I können aus dem jeweiligen Reaktionsgemisch nach gängigen Verfahren wie Kristallisation, Sublimation, Chromatographie oder Destillation abgetrennt und gewünschtenfalls gereinigt werden, je nach den Umständen des Einzelfalles können die Zwischenstufen aber auch ohne Zwischenisolierung weiter umgesetzt werden. In den erhaltenen Verbindungen können zudem funktionelle Gruppen umgewandelt werden. Beispielsweise können Thioethergruppierungen durch Oxidation mit einer Peroxyverbindung wie 3-Chlorperbenzoesäure oder Monoperoxyphthalsäure oder Wasserstoffperoxid in Sulfone oder Sulfoxide umgewandelt werden, oder es können Carbonsäureestergruppen zu den Carbonsäuren verseift werden.

Alle Reaktionen zur Synthese der Verbindungen der Formel I sind dem Fachmann an sich wohlbekannt und können unter Standardbedingungen nach oder analog zu Literaturvorschriften durchgeführt werden, wie sie zum Beispiel in Houben-Weyl, Methoden der Organischen Chemie, Thieme-Verlag, Stuttgart, oder Organic Reactions, John Wiley & Sons, New York, beschrieben sind. Je nach den Gegebenheiten des Einzelfalls kann es bei der Synthese der Verbindungen der Formel I zur Vermeidung von Nebenreaktionen auch vorteilhaft oder notwendig sein, bestimmte funktionelle Gruppen vorübergehend durch die Einführung von Schutzgruppen zu blockieren und später dann wieder freizusetzen oder funktionelle Gruppen zunächst in Form von Vorstufen einzusetzen, aus denen in einem späteren Schritt dann die gewünschte funktionelle Gruppe erzeugt wird. Solche Synthesestrategien und die für den Einzelfall geeigneten Schutzgruppen oder Vorstufen sind dem Fachmann bekannt. Die Ausgangsamidine der Formel II oder deren Salze, die Oxoester der Formel III und die Amine der Formel VI sind kommerziell erhältlich oder können nach oder analog zu bekannten Verfahren hergestellt werden.

Die erfindungsgemäßen Verbindungen der Formel I bewirken über die Aktivierung der löslichen Guanylatcyclase (sGC) eine Erhöhung der cGMP-Konzentration und sind deshalb wertvolle Agenzien zur Therapie und Prophylaxe von Krankheiten, die mit einem niedrigen oder erniedrigten cGMP-Spiegel verbunden sind oder durch einen solchen verursacht werden oder zu deren Therapie oder Prophylaxe eine Erhöhung des vorhandenen cGMP-Spiegels angestrebt wird. Die Aktivierung der sGC durch die Verbindungen der Formel I kann zum Beispiel in dem unten beschriebenen Aktivitätsassay untersucht werden.

Krankheiten und pathologische Zustände, die mit einem niedrigen cGMP-Spiegel verbunden sind oder bei denen eine Erhöhung des cGMP-Spiegels angestrebt wird und zu deren Therapie und Prophylaxe Verbindungen der Formel I eingesetzt werden können, sind zum Beispiel Herz-Kreislauf-Erkrankungen wie endotheliale Dysfunktion, diastolische Dysfunktion, Atherosklerose, Bluthochdruck, stabile und instabile Angina pectoris, Thrombosen, Restenosen, Myocardinfarkt, Schlaganfälle, Herzinsuffizienz oder Pulmonalhypertonie, oder zum Beispiel erektile Dysfunktion, Asthma bronchiale, chronische Niereninsuffizienz und Diabetes. Verbindungen der Formel I können darüber hinaus eingesetzt werden bei Leberzirrhose sowie zur Verbesserung einer eingeschränkten Lernfähigkeit oder Gedächtnisleistung.

Die Verbindungen der Formel I und ihre physiologisch verträglichen Salze können somit am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind daher auch die Verbindungen der Formel I und ihre physiologisch verträglichen Salze zur Verwendung als Arzneimittel, ihre Verwendung zur Normalisierung eines gestörten cGMP-Haushalts und insbesondere ihre Verwendung in der Therapie und Prophylaxe der oben genannten Krankheitsbilder, sowie ihre Verwendung zur Herstellung von Medikamenten dafür. Weiterhin sind Gegenstand der vorliegenden Erfindung pharmazeutische Präparate (oder pharmazeutische Zusammensetzungen), die eine wirksame Dosis mindestens einer Verbindung der Formel I und/oder eines physiologisch verträglichen Salzes davon als aktiven Bestandteil und einen pharmazeutisch verträglichen Träger, das heißt einen oder mehrere übliche pharmazeutisch verträgliche Trägerstoffe und/oder Hilfsstoffe (oder Zusatzstoffe), enthalten.

Die erfindungsgemäßen Arzneimittel können oral, zum Beispiel in Form von Pillen, Tabletten, Filmtabletten, Dragees, Granulaten, Hart- und Weichgelatinekapseln, wäßrigen, alkoholischen oder öligen Lösungen, Sirupen, Emulsionen oder Suspensionen, oder rektal, zum Beispiel in Form von Suppositorien, verabreicht werden. Die Verabreichung kann aber auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Tinkturen, Sprays oder transdermalen therapeutischen Systemen, oder die inhalative Applikation in Form von Nasalsprays oder Aerosolmischungen, oder zum Beispiel Mikrokapseln, Implantate oder Rods. Die bevorzugte Applikationsform hängt zum Beispiel von der zu behandelnden Krankheit und ihrer Stärke ab.

Die pharmazeutischen Präparate enthalten normalerweise 0.2 bis 500 mg, vorzugsweise 1 bis 200 mg, Wirkstoff der Formel I und/oder dessen physiologisch verträgliche Salze, je nach der Art des Präparats und der vorgesehenen Verwendung kann die Menge des enthaltenen Wirkstoffs aber auch größer sein. Die Herstellung der pharmazeutischen Präparate kann in an sich bekannter Weise erfolgen. Dazu werden ein oder mehrere Verbindungen der Formel I und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen mit therapeutischer oder prophylaktischer Wirkung in eine geeignete Verabreichungsform bzw. Dosierungsform gebracht, die dann als Arzneimittel in der Humanmedizin oder Veterinärmedizin verwendet werden kann. Die pharmazeutischen Präparate enthalten normalerweise 0.5 bis 90 Gewichtsprozent der Verbindungen der Formel I und/oder ihrer physiologisch verträglichen Salze.

Für die Herstellung beispielsweise von Pillen, Tabletten, Dragees und Hartgelatinekapseln kann man Lactose, Stärke, zum Beispiel Maisstärke, oder Stärkederivate, Talk, Stearinsäure oder deren Salze, etc. verwenden. Trägerstoffe für Weichgelatinekapseln und Suppositorien sind zum Beispiel Fette, Wachse, halbfeste und flüssige Polyole, natürliche oder gehärtete Öle etc. Als Trägerstoffe für die Herstellung von Lösungen, zum Beispiel Injektionslösungen, oder von Emulsionen oder Sirupen eignen sich beispielsweise Wasser, physiologische Kochsalzlösung, Alkohole wie Ethanol, Glycerin, Polyole, Saccharose, Invertzucker, Glucose, Mannit, pflanzliche Öle etc. Die Verbindungen der Formel I und ihre physiologisch verträglichen Salze können auch lyophilisiert werden und die erhaltenen Lyophilisate zum Beispiel zur Herstellung von Injektionspräparaten oder Infusionspräparaten verwendet werden. Als Trägerstoffe für Mikrokapseln, Implantate oder Rods eignen sich zum Beispiel Mischpolymerisate aus Glykolsäure und Milchsäure.

Die pharmazeutischen Präparate können neben den Wirkstoffen und Trägerstoffen noch übliche Hilfsstoffe oder Zusatzstoffe enthalten, zum Beispiel Füllstoffe, Spreng-, Binde-, Gleit-, Netz-, Stabilisierungs-, Emulgier-, Dispergier-, Konservierungs-, Süß-, Färbe-, Geschmacks-, Aromatisierungs-, Dickungs-, Verdünnungsmittel, Puffersubstanzen, Lösungsmittel, Lösungsvermittler, Mittel zur Erzielung eines Depoteffekts, Salze zur Veränderung des osmotischen Drucks, Überzugsmittel oder Antioxidantien.

Die Dosierung des zu verabreichenden Wirkstoffs der Formel I und/oder eines physiologisch verträglichen Salze davon hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter,

Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel I weitere Wirkstoffe verabreicht werden.

Im allgemeinen ist eine Tagesdosis von etwa 0.01 bis 100 mg/kg, vorzugsweise 0.1 bis 10 mg/kg, insbesondere 0.3 bis 5 mg/kg (jeweils mg pro kg Körpergewicht) bei Verabreichung an einen ca. 75 kg schweren Erwachsenen zur Erzielung der angestrebten Wirkung angemessen. Die Tagesdosis kann in einer Einzeldosis verabreicht werden oder, insbesondere bei der Applikation größerer Mengen, in mehrere, zum Beispiel zwei, drei oder vier Einzeldosen aufgeteilt werden.

Gegebenenfalls kann es, je nach individuellem Verhalten, erforderlich werden, von der angegebenen Tagesdosis nach oben oder nach unten abzuweichen.

Die Verbindungen der Formel I aktivieren die lösliche Guanylatcyclase. Aufgrund dieser Eigenschaft können sie außer als Arzneimittelwirkstoffe in der Humanmedizin und Veterinärmedizin auch als wissenschaftliches Tool oder als Hilfsmittel für biochemische Untersuchungen eingesetzt werden, bei denen eine derartige Beeinflussung der Guanylatcyclase beabsichtigt ist, sowie für diagnostische Zwecke, zum Beispiel in der in vitro-Diagnostik von Zellproben oder Gewebsproben. Ferner können die Verbindungen der Formel I und ihre Salze, wie bereits oben erwähnt, als Zwischenprodukte zur Herstellung weiterer Arzneimittelwirkstoffe dienen.

Die nachfolgenden Beispiele erläutern die Erfindung, ohne sie einzuschränken.

Beispiele

Beispiel 1

2-(4-Chlorphenyl)-4-hydroxy-6-isopropyl-pyrimidin

Eine Mischung aus 19.1 g 4-Chlorbenzamidin-hydrochlorid, 15.8 g 4-Methyl-3-oxopentansäureethylester, 11.2 g Kalium-tert-butylat und 200 ml Ethanol wurde 2 Stunden unter Rückfluß erhitzt. Nach dem Abkühlen auf Raumtemperatur wurde der Feststoff abgesaugt, mit Wasser und mit wenig Ethanol gewaschen und im Vakuum bei 40°C getrocknet. Ausbeute: 12.5 g.

Schmp.: 164°C

Analog wurden hergestellt:

Beispiel 2

2-(4-Chlorphenyl)-4-hydroxy-6-trifluormethyl-pyrimidin; Schmp.: 258°C

Beispiel 3

2-(4-Chlorphenyl)-6-tert-butyl-4-hydroxy-pyrimidin; Schmp.: 193°C

Beispiel 4

2-(4-Chlorphenyl)-4-hydroxy-6-phenyl-pyrimidin; Schmp.: 306°C

Beispiel 5

2-(4-Methylphenyl)-4-hydroxy-6-isopropyl-pyrimidin; Schmp.: 164°C

Beispiel 6

2-(3,5-Dichlorphenyl)-4-hydroxy-6-isopropyl-pyrimidin; Schmp.: 203°C

Beispiel 7

2-(4-Aminocarbonyl-phenyl)-4-hydroxy-6-isopropyl-pyrimidin; Schmp.: 294°C

Beispiel 8

4-Chlor-2-(4-chlorphenyl)-6-isopropyl-pyrimidin

Die Mischung aus 12 g 2-(4-Chlorphenyl)-4-hydroxy-6-isopropyl-pyrimidin und 35 ml Phosphoroxychlorid wurde 3 Stunden unter Rühren auf 90°C erhitzt. Der größte Teil des Phosphoroxychloridüberschusses wurde im Vakuum abdestilliert, der Rückstand wurde auf 100 ml Eiswasser gegeben und verrührt. Der sich bildende feste weiße Niederschlag wurde abgesaugt und im Vakuum bei Raumtemperatur getrocknet. Ausbeute: 11.4 g.
Schmp.: 74°C

Analog wurden hergestellt:

Beispiel 9

4-Chlor-2-(4-chlorphenyl)-6-trifluormethyl-pyrimidin; Schmp.: 76°C

Beispiel 10

4-Chlor-2-(4-chlorphenyl)-6-tert-butyl-pyrimidin; Schmp.: 93°C

Beispiel 11

4-Chlor-2-(4-chlorphenyl)-6-phenyl-pyrimidin; Schmp.: 127°C

Beispiel 12

4-Chlor-2-(4-methylphenyl)-6-isopropyl-pyrimidin; Schmp.: Öl

Beispiel 13

4-Chlor-2-(3,5-dichlorphenyl)-6-isopropyl-pyrimidin; Schmp.: 59°C

Beispiel 14

4-Chlor-2-(4-cyanphenyl)-6-isopropyl-pyrimidin vom Schmp. 114°C wurde in analoger Reaktion ausgehend von 2-(4-Aminocarbonylphenyl)-4-hydroxy-6-isopropyl-pyrimidin erhalten.

Beispiel 15

2-(4-Chlorphenyl)-6-isopropyl-4-((2,2,6,6-tetramethyl-piperidin-4-yl)-amino)-pyrimidin-dihydrochlorid
Eine Mischung aus 534 mg 4-Chlor-2-(4-chlorphenyl)-6-isopropyl-pyrimidin und 1.8 g 4-Amino-2,2,6,6-tetramethyl-piperidin wurde 2 Stunden unter Rühren auf 150°C erhitzt. Nach dem Erkalten wurden 20 ml Wasser zugefügt und die Mischung bei Raumtemperatur gerührt. Der weiße Niederschlag wurde abgesaugt, im Vakuum getrocknet und in 20 ml Ethylacetat aufgenommen. Durch Zusatz von Chlorwasserstoff wurde die Titelverbindung gefällt, abgesaugt und im Vakuum getrocknet. Ausbeute: 0.8 g.
Schmp.: 359°C

Beispiel 16

2-(4-Chlorphenyl)-6-isopropyl-4-morpholino-pyrimidin

Eine Mischung aus 267 mg 4-Chlor-2-(4-chlorphenyl)-6-isopropyl-pyrimidin und 522 mg Morpholin wurde 2 Stunden auf 130°C erhitzt. Nach dem Erkalten wurden 20 ml Wasser zugefügt, die Mischung gerührt, der Feststoff abgesaugt und im Vakuum bei 50°C getrocknet. Ausbeute: 0.28 g.
Schmp.: 123°C

Analog den Beispielen 15 und 16 wurden die folgenden Verbindungen der Formel I hergestellt. Wenn in der Spalte "Schmp." eine Säure angegeben ist, wurde die Verbindung in Form des Säureadditionssalzes mit der angegebenen Säure erhalten. Die Angabe "2HCl" bedeutet, daß die Verbindung als Dihydrochlorid erhalten wurde.

| Bsp. Nr. | $R^4$ | $R^3$ | $R^1R^2N$ | Schmp. (°C) |
|---|---|---|---|---|
| 17 | $CF_3$ | 4-Chlorphenyl | (3-Phenyl-propyl)-amino | Öl |
| 18 | $CF_3$ | 4-Chlorphenyl | (2-Ethylthio-ethyl)-amino | 114 (HCl) |
| 19 | $CF_3$ | 4-Chlorphenyl | (1-Benzyl-piperidin-4-yl)-amino | 128 (2HCl) |
| 20 | $CF_3$ | 4-Chlorphenyl | 4-(2-Hydroxy-ethyl)-piperazino | 119 |
| 23 | Isopropyl | 4-Methylphenyl | (3-Methoxy-propyl)-amino | Öl |
| 24 | Isopropyl | 4-Methylphenyl | Cyclopentyl-amino | 66 |
| 25 | Isopropyl | 4-Methylphenyl | (trans-4-Hydroxy-cyclohexyl)-amino | Öl |

(fortgesetzt)

| Bsp. Nr. | $R^4$ | $R^3$ | $R^1R^2N$ | Schmp. (°C) |
|---|---|---|---|---|
| 26 | Isopropyl | 4-Chlorphenyl | (3-Methoxy-propyl)-amino | Öl |
| 27 | Isopropyl | 4-Chlorphenyl | 4-Methyl-piperazino | 292 (2HCl) |
| 28 | Isopropyl | 4-Chlorphenyl | Piperidino | 75 |
| 29 | Isopropyl | 4-Chlorphenyl | Pyrrolidino | 215 (HCl) |
| 30 | Isopropyl | 4-Chlorphenyl | Thiomorpholino | 215 (HCl) |
| 31 | Isopropyl | 4-Chlorphenyl | (2-(3-Methoxy-phenyl)-ethyl)-amino | 213 (HCl) |
| 32 | isopropyl | 4-Chlorphenyl | Butyl-amino | Öl |
| 33 | Isopropyl | 4-Chlorphenyl | Di-ethyl-amino | Öl |
| 34 | Isopropyl | 4-Chlorphenyl | Di-butyl-amino | 165 (HCl) |
| 35 | Isopropyl | 4-Chlorphenyl | Di-propyl-amino | 176 (HCl) |
| 36 | Isopropyl | 4-Chlorphenyl | Di-allyl-amino | 118 |
| 37 | Isopropyl | 4-Chlorphenyl | Di-(2-methoxy-ethyl)-amino | 127 (HCl) |
| 38 | Isopropyl | 4-Chlorphenyl | Perhydroazepin-1-yl | 68 |
| 39 | Isopropyl | 4-Chlorphenyl | Benzyl-amino | 108 |
| 40 | Isopropyl | 4-Chlorphenyl | (2-Methoxy-ethyl)-amino | 152 (HCl) |
| 41 | Isopropyl | 4-Chlorphenyl | (2-Ethylmercapto-ethyl)-amino | 148 (HCl) |
| 42 | Isopropyl | 4-Chlorphenyl | (3-Morpholino-propyl)-amino | 245 (2HCl) |
| 43 | Isopropyl | 4-Chlorphenyl | N-(Ethyl)-N-(benzyl)-amino | Öl |
| 44 | Isopropyl | 4-Chlorphenyl | 4-Aminocarbonyl-piperidino | 189 |
| 45 | Isopropyl | 4-Chlorphenyl | 1,3-Thiazolidin-3-yl | 77 |
| 46 | Isopropyl | 4-Chlorphenyl | 4-(Dimethylamino-sulfonyl)-piperazino | 150 |
| 47 | Isopropyl | 4-Chlorphenyl | 4-Benzyl-piperazino | 265 (2HCl) |
| 48 | Isopropyl | 4-Chlorphenyl | 4-((Isopropylamino-carbonyl)-methyl)-piperazino | 133 |
| 49 | tert-Butyl | 4-Chlorphenyl | 4-Methyl-piperazino | 122 |
| 50 | tert-Butyl | 4-Chlorphenyl | (2-Methoxy-ethyl)-amino | 94 |
| 51 | tert-Butyl | 4-Chlorphenyl | (3-Pyridyl-methyl)-amino | 143 |
| 52 | tert-Butyl | 4-Chlorphenyl | Morpholino | 136 |
| 53 | tert-Butyl | 4-Chlorphenyl | 4-(Dimethylamino-sulfonyl)-piperazino | 168 |
| 54 | tert-Butyl | 4-Chlorphenyl | (2,2,6,6-Tetramethylpiperidin-4-yl)-amino | 142 |
| 55 | Phenyl | 4-Chlorphenyl | Morpholino | 193 |
| 56 | Phenyl | 4-Chlorphenyl | 4-Methyl-piperazino | 167 |
| 57 | Phenyl | 4-Chlorphenyl | (3-Pyridyl-methyl)-amino | 130 |
| 58 | Phenyl | 4-Chlorphenyl | (3-(Imidazol-1-yl)-propyl)-amino | 154 |
| 59 | Phenyl | 4-Chlorphenyl | (2-(3-Methoxy-phenyl)-ethyl)-amino | 103 (HCl) |
| 60 | Phenyl | 4-Chlorphenyl | 4-Carboxy-1,3-thiazolidin-3-yl | 113 |
| 63 | Phenyl | 4-Chlorphenyl | Di-ethyl-amino | 132 |
| 64 | Phenyl | 4-Chlorphenyl | Butyl-amino | 95 (HCl) |
| 65 | Phenyl | 4-Chlorphenyl | Thiomorpholino | 175 |
| 66 | tert-Butyl | 4-Chlorphenyl | Thiomorpholino | 119 |
| 69 | Phenyl | 4-Chlorphenyl | Di-propyl-amino | 72 |
| 70 | Isopropyl | 4-Chlorphenyl | Cyclopropyl-amino | Öl |
| 71 | $CF_3$ | 4-Chlorphenyl | (3-Pyridyl-methyl)-amino | 181 |
| 72 | Isopropyl | 4-Chlorphenyl | 3,3-Dimethyl-piperidino | Öl |
| 73 | $CF_3$ | 4-Chlorphenyl | 4-Methyl-piperazino | 108 |
| 74 | $CF_3$ | 4-Chlorphenyl | Morpholino | 184 |
| 75 | tert-Butyl | 4-Chlorphenyl | Perhydroazepin-1-yl | 151 |
| 76 | tert-Butyl | 4-Chlorphenyl | 4-Aminocarbonyl-piperidino | 164 |
| 77 | Isopropyl | 3,5-Dichlorphenyl | (trans-4-Hydroxy-cyclohexyl)-amino | 174 |

(fortgesetzt)

| Bsp. Nr. | $R^4$ | $R^3$ | $R^1R^2N$ | Schmp. (°C) |
|---|---|---|---|---|
| 78 | Isopropyl | 3,5-Dichlorphenyl | (2-Hydroxy-ethyl)-amino | 88 |
| 79 | Isopropyl | 3,5-Dichlorphenyl | Butyl-amino | 190 (HCl) |
| 80 | Isopropyl | 3,5-Dichlorphenyl | Di-ethyl-amino | Öl |
| 81 | Isopropyl | 3,5-Dichlorphenyl | Morpholino | 138 |
| 82 | Isopropyl | 3,5-Dichlorphenyl | Thiomorpholino | 130 |
| 83 | Isopropyl | 3,5-Dichlorphenyl | 4-Methyl-piperazino | 123 |
| 84 | Isopropyl | 3,5-Dichlorphenyl | Di-propyl-amino | Öl |
| 85 | Isopropyl | 4-Methylphenyl | Di-propyl-amino | Öl |
| 86 | Isopropyl | 4-Methylphenyl | Di-ethyl-amino | 180 (HCl) |
| 87 | Isopropyl | 4-Methylphenyl | (3-Hydroxy-propyl)-amino | 86 |
| 88 | Isopropyl | 4-Methylphenyl | Butyl-amino | Öl |
| 89 | Isopropyl | 4-Methylphenyl | Morpholino | 95 |
| 90 | Isopropyl | 4-Methylphenyl | Thiomorpholino | 107 |
| 91 | Isopropyl | 4-Methylphenyl | 4-Methyl-piperazino | 70 |
| 92 | Isopropyl | 4-Chlorphenyl | N-(Ethyl)-N-(butyl)-amino | Öl |
| 93 | Isopropyl | 4-Chlorphenyl | N-(Methyl)-N-(butyl)-amino | Öl |
| 94 | Isopropyl | 4-Chlorphenyl | 4-(2-Pyridyl)-piperazino | 166 |
| 95 | $CF_3$ | 4-Chlorphenyl | 4-(2-Pyridyl)-piperazino | 174 |
| 96 | Isopropyl | 4-Chlorphenyl | cis/trans-2,6-Dimethyl-morpholino | Öl |
| 97 | Isopropyl | 4-Methylphenyl | cis-2,6-Dimethyl-morpholino | Öl |
| 98 | Isopropyl | 4-Methylphenyl | Di-(2-methoxy-ethyl)-amino | Öl |
| 99 | Isopropyl | 4-Methylphenyl | 4-Aminocarbonyl-piperidino | 192 |
| 100 | Isopropyl | 4-Methylphenyl | Perhydroazepin-1-yl | Öl |
| 101 | tert-Butyl | 4-Chlorphenyl | cis-2,6-Dimethyl-morpholino | 117 |
| 102 | tert-Butyl | 4-Chlorphenyl | (3-Methoxy-propyl)-amino | Öl |
| 103 | tert-Butyl | 4-Chlorphenyl | Di-(2-methoxy-ethyl)-amino | Öl |
| 104 | Isopropyl | 4-Chlorphenyl | cis-2,6-Dimethyl-morpholino | 114 |
| 105 | Isopropyl | 4-Chlorphenyl | (2-Diisopropylamino-ethyl)-amino | 219 (2HCl) |
| 106 | Isopropyl | 4-Chlorphenyl | 4-(2-Hydroxy-ethyl)-piperazino | 227 (2HCl) |
| 107 | Isopropyl | 4-Chlorphenyl | (1-Benzyl-piperidin-4-yl)-amino | 250 (2HCl) |
| 108 | Phenyl | 4-Chlorphenyl | cis/trans-2,6-Dimethyl-morpholino | 187 |
| 109 | Phenyl | 4-Chlorphenyl | (3-Methoxy-propyl)-amino | Öl |
| 110 | Phenyl | 4-Chlorphenyl | Di-(2-methoxy-ethyl)-amino | Öl |
| 111 | Phenyl | 4-Chlorphenyl | 4-Aminocarbonyl-piperidino | 204 |
| 112 | Phenyl | 4-Chlorphenyl | Perhydroazepin-1-yl | 126 |
| 113 | Isopropyl | 4-Cyanphenyl | (4-Hydroxy-butyl)-amino | 93 |
| 114 | Isopropyl | 4-Cyanphenyl | (3-Methoxy-propyl)-amino | 70 |
| 115 | Isopropyl | 4-Cyanphenyl | Butyl-amino | 89 |
| 116 | Isopropyl | 4-Cyanphenyl | Cyclopentyl-amino | 141 |
| 117 | Isopropyl | 4-Cyanphenyl | (4-Hydroxy-cyclohexyl)-amino | 101 |
| 118 | Isopropyl | 4-Cyanphenyl | (3-Pyridyl-methyl)-amino | 149 |
| 119 | Isopropyl | 4-Cyanphenyl | Di-propyl-amino | 80 |
| 120 | Isopropyl | 4-Cyanphenyl | Perhydroazepin-1-yl | 117 |
| 121 | Isopropyl | 4-Cyanphenyl | Morpholino | 224 |
| 122 | Isopropyl | 4-Cyanphenyl | 4-Methyl-piperazino | 152 |
| 123 | Isopropyl | 2-Methylthiazol-4-yl | Di-propyl-amino | Öl |
| 124 | Isopropyl | 4-Chlorphenyl | Cyclopentyl-amino | 82 |
| 125 | Isopropyl | 4-Chlorphenyl | (trans-4-Hydroxy-cyclohexyl)-amino | 138 |

(fortgesetzt)

| Bsp. Nr. | $R^4$ | $R^3$ | $R^1R^2N$ | Schmp. (°C) |
|---|---|---|---|---|
| 126 | Isopropyl | 4-Chlorphenyl | (trans-4-Amino-cyclohexyl)-amino | 128 |
| 127 | Isopropyl | 4-Chlorphenyl | (cis/trans-4-Hydroxy-cyclohexyl)-amino | Öl |
| 128 | Isopropyl | 4-Chlorphenyl | (4-Methyl-cyclohexyl)-amino | Öl |
| 129 | Isopropyl | 4-Chlorphenyl | N-(Cyclohexyl)-N-(methyl)-amino | 88 |
| 130 | Isopropyl | 4-Chlorphenyl | (2-Isopropyl-5-methyl-cyclohexyl)-amino | Öl |
| 131 | Isopropyl | 4-Chlorphenyl | (trans-2-Hydroxy-cyclohexyl)-amino | Öl |
| 132 | tert-Butyl | 4-Chlorphenyl | Cyclopentyl-amino | 89 |
| 133 | tert-Butyl | 4-Chlorphenyl | (trans-4-Hydroxy-cyclohexyl)-amino | 173 |
| 134 | $CF_3$ | 4-Chlorphenyl | Cyclopentyl-amino | 99 |
| 135 | Phenyl | 4-Chlorphenyl | (trans-4-Hydroxy-cyclohexyl)-amino | 95 |
| 136 | Isopropyl | 4-Chlorphenyl | 4-Hydroxy-piperidino | 121 |
| 137 | Isopropyl | 4-Chlorphenyl | (4-Hydroxy-butyl)-amino | Öl |
| 138 | Isopropyl | 4-Chlorphenyl | (Benzimidazol-2-yl-methyl)-amino | 112 |
| 139 | Isopropyl | 4-Chlorphenyl | Cyclobutyl-amino | 70 |
| 140 | Isopropyl | 4-Chlorphenyl | Cyclononyl-amino | Öl |
| 141 | Isopropyl | 4-Chlorphenyl | 3-Diethylaminocarbonyl-piperidino | Öl |
| 142 | Isopropyl | 4-Chlorphenyl | ((R)-1-Phenyl-ethyl)-amino | Öl |
| 143 | Isopropyl | 4-Chlorphenyl | ((S)-1-Phenyl-ethyl)-amino | Öl |

Beispiel 144

2-(4-Chlorphenyl)-6-isopropyl-4-(1-oxo-thiomorpholino)-pyrimidin

0.25 g 2-(4-Chlorphenyl)-6-isopropyl-4-thiomorpholino-pyrimidin wurden in 1 ml Eisessig gelöst und mit 0.068 ml einer 35 %igen Wasserstoffperoxidlösung versetzt. Nach 2 Stunden wurde mit 20 ml Wasser verdünnt und das Produkt mit Ethylacetat extrahiert. Die Ethylacetatphase wurde zweimal mit 10 ml Wasser ausgeschüttelt und die organische Phase nach dem Trocknen über Natriumsulfat eingeengt. Der Rückstand wurde aus Isopropanol umkristallisiert. Ausbeute: 0.18 g.
Schmp.: 171°C

Analog wurden die folgenden Sulfoxide und Sulfone hergestellt:

Beispiel 145

2-(4-Chlorphenyl)-6-isopropyl-4-(1,1-dioxo-thiomorpholino)-pyrimidin; Schmp.: 226°C

Beispiel 146

2-(4-Chlorphenyl)-6-isopropyl-4-(1-oxo-1,3-thiazolidin-1-yl)-pyrimidin; Schmp.: 128°C

Beispiel 147

2-(4-Chlorphenyl)-4-((2-ethylsulfinyl-ethyl)-amino)-6-isopropyl-pyrimidin; Schmp.: 103°C

Beispiel 148

6-Isopropyl-2-(4-methylphenyl)-4-(1-oxo-thiomorpholino)-pyrimidin; Schmp.: 152°C

Beispiel 149

2-(3,5-Dichlor-phenyl)-6-isopropyl-4-(1-oxo-thiomorpholino)-pyrimidin; Schmp.: 187°C

Pharmakologische Untersuchungen

Aktivierung der löslichen Guanylatcyclase

Die Aktivierung der löslichen Guanylatcyclase (sGC), die die Umwandlung von Guanosintriphosphat (GTP) in cyclisches Guanosinmonophosphat (cGMP) und Pyrophosphat katalysiert, durch die erfindungsgemäßen Verbindungen wurde mit Hilfe eines Enzym-Immuno-Assays (EIA) der Firma Amersham quantifiziert. Dazu wurden die Prüfsubstanzen zunächst mit sGC in Mikrotiterplatten inkubiert und dann die Menge des entstandenen cGMP bestimmt.

Die eingesetzte sGC war aus Rinderlunge isoliert worden (siehe Methods in Enzymology, Band 195, S. 377). Die Testlösungen (100 µl pro well) enthielten 50 mM Triethanolamin(TEA)-Puffer (pH 7.5), 3 mM $MgCl_2$, 3 mM reduziertes Glutathion (GSH), 0.1 mM GTP, 1 mM 3-Isobutyl-1-methylxanthin (IBMX), geeignet verdünnte Enzymlösung sowie die Prüfsubstanz bzw. bei den Kontrollversuchen Lösungsmittel. Die Prüfsubstanzen wurden in Dimethylsulfoxid (DMSO) gelöst und die Lösung mit DMSO/Wasser verdünnt, so daß die Endkonzentration c an Prüfsubstanz im Testansatz 50 µM betrug. Die DMSO-Konzentration im Testansatz betrug 5 % (v/v). Die Reaktion wurde durch Zugabe der sGC gestartet. Der Reaktionsmix wurde für 15 bis 20 Minuten bei 37°C inkubiert und dann durch Eiskühlung und Zugabe des Stop-Reagenz (50 mM EDTA, pH 8.0) gestoppt. Ein Aliquot von 50 µl wurde entnommen und zur Bestimmung des cGMP-Gehaltes mit dem Acetylierungs-Protokoll des Amersham-cGMP-EIA-Kits eingesetzt. Die Absorption der Proben wurde bei 450 nm (Referenz Wellenlänge 620 nm) in einem Mikrotiterplatten-Lesegerät gemessen. Die cGMP-Konzentration wurde über eine Eichkurve ermittelt, die unter denselben Versuchsbedingungen erhalten wurde. Die Aktivierung der sGC durch eine Prüfsubstanz wird angegeben als n-fache Stimulation der basalen Enzymaktivität, die bei den Kontrollversuchen (mit Lösungsmittel statt Prüfsubstanz) gefunden wurde (berechnet nach der Formel

$$\text{n-fache Stimulierung} = [\text{cGMP}]_{\text{Prüfsubstanz}} / [\text{cGMP}]_{\text{Kontrolle}}).$$

Es wurden folgende Ergebnisse erhalten:

| Verbindung des Beispiels Nr. | n-fache Stimulation bei c = 50 µM |
|---|---|
| 23 | >8 |
| 25 | 28 |
| 29 | >4 |
| 30 | >4 |
| 32 | >4 |
| 33 | >16 |
| 34 | >4 |
| 35 | >16 |

(fortgesetzt)

| Verbindung des Beispiels Nr. | n-fache Stimulation bei c = 50 µM |
|---|---|
| 36 | >8 |
| 38 | >8 |
| 43 | >4 |
| 44 | >4 |
| 45 | >4 |
| 52 | >8 |
| 63 | >8 |
| 66 | >4 |
| 69 | >4 |
| 77 | 30 |
| 79 | >4 |
| 80 | >16 |
| 81 | >4 |
| 82 | >4 |
| 84 | >16 |
| 85 | >16 |
| 86 | >16 |
| 88 | >8 |
| 89 | >8 |
| 90 | >16 |
| 97 | >16 |
| 98 | >8 |
| 99 | >4 |
| 100 | >8 |
| 112 | >8 |
| 124 | >16 |
| 125 | 32 |
| 133 | >16 |
| 137 | >16 |

**Patentansprüche**

1. Verbindungen der Formel I,

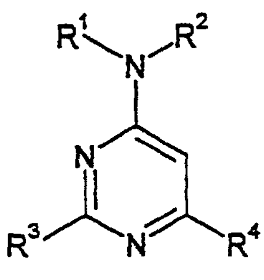

I

in der

$R^1$ für ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-S(O)$_m$-, $R^5R^6N$ und Aryl substituiert sein kann, ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, oder den Rest eines 5-gliedrigen bis 7-gliedrigen gesättigten heterocyclischen Ringes, der

ein oder zwei gleiche oder verschiedene Hetero-Ringglieder aus der Reihe O, $NR^7$ und $S(O)_m$ enthält und der durch einen oder mehrere gleiche oder verschiedene Substituenten aus der Reihe $(C_1-C_4)$-Alkyl und Aryl-$(C_1-C_4)$-alkyl- substituiert sein kann, steht;

und

$R^2$ für Wasserstoff, $(C_1-C_8)$-Alkyl, das durch einen oder mehrere gleiche oder verschiedene Substituenten aus der Reihe Hydroxy, $(C_1-C_4)$-Alkoxy, $(C_1-C_4)$-Alkyl-$S(O)_m$-, $R^5R^6N$ und Aryl substituiert sein kann, $(C_3-C_9)$-Cycloalkyl, das durch einen oder mehrere gleiche oder verschiedene Substituenten aus der Reihe $(C_1-C_4)$-Alkyl, Hydroxy und Amino substituiert sein kann, oder den Rest eines 5-gliedrigen bis 7-gliedrigen gesättigten heterocyclischen Ringes, der ein oder zwei gleiche oder verschiedene Hetero-Ringglieder aus der Reihe 0, $NR^7$ und $S(O)_m$ enthält und der durch einen oder mehrere gleiche oder verschiedene Substituenten aus der Reihe $(C_1-C_4)$-Alkyl und Aryl-$(C_1-C_4)$-alkyl- substituiert sein kann, steht;

oder

$R^1R^2N$ für einen über ein Ringstickstoffatom gebundenen Rest eines 5-gliedrigen bis 7-gliedrigen gesättigten heterocyclischen Ringes steht, der neben dem die Reste $R^1$ und $R^2$ tragenden Stickstoffatom ein weiteres Hetero-Ringglied aus der Reihe O, $NR^7$ und $S(O)_m$ enthalten kann und der durch einen oder mehrere gleiche oder verschiedene Substituenten aus der Reihe $(C_1-C_4)$-Alkyl, Hydroxy, $(C_1-C_4)$-Alkoxy, $R^8R^9N$, Hydroxycarbonyl, $(C_1-C_4)$ -Alkoxycarbonyl und $R^8R^9N$-CO- substituiert sein kann;

$R^3$ für Phenyl steht, das durch einen oder mehrere gleiche oder verschiedene Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-Alkyl, $(C_1-C_2)$-Alkylendioxy, $NH_2$, -NH-$(C_1-C_4)$-Alkyl, -N($(C_1-C_4)$-Alkyl$)_2$, -NH-CHO, -NH-CO-$(C_1-C_4)$-Alkyl, -CN, -CO-$NH_2$, -CO-NH-$(C_1-C_4)$-Alkyl, -CO-N($(C_1-C_4)$-Alkyl$)_2$, -CO-OH. -CO-O-$(C_1-C_4)$-Alkyl, -CHO und -CO-$(C_1-C_4)$-Alkyl substituiert sein kann;

$R^4$ für $(C_2-C_5)$-Alkyl, Trifluormethyl oder Phenyl, das durch einen oder mehrere gleiche oder verschiedene Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-Alkyl, $(C_1-C_2)$-Alkylendioxy, $NH_2$, -NH-$(C_1-C_4)$-Alkyl, -N($(C_1-C_4)$-Alkyl$)_2$, -NH-CHO, -NH-CO-$(C_1-C_4)$-Alkyl, -CN, -CO-$NH_2$, -CO-NH-$(C_1-C_4)$-Alkyl, -CO-N($(C_1-C_4)$-Alkyl$)_2$, -CO-OH, CO-O-$(C_1-C_4)$-Alkyl, -CHO und -CO-$(C_1-C_4)$-Alkyl substituiert sein kann, steht;

$R^5$ und $R^6$ für gleiche oder verschiedene Reste aus der Reihe Wasserstoff und $(C_1-C_4)$-Alkyl stehen oder die Gruppe $R^5R^6N$ für einen über ein Ringstickstoffatom gebundenen Rest eines 5-gliedrigen bis 7-gliedrigen gesättigten oder ungesättigten heterocyclischen Ringes steht, der neben dem die Reste $R^5$ und $R^6$ tragenden Stickstoffatom als weiteres Hetero-Ringglie noch ein Sauerstoffatom, eine Gruppe $S(O)_m$ oder ein Stickstoffatom enthalten kann und der an Ringkohlenstoffatomen einen oder mehrere gleiche oder verschiedene Substituenten aus der Reihe $(C_1-C_4)$-Alkyl, Hydroxy und Amino tragen kann und an einem Ringstickstoffatom einen Rest $R^7$ tragen kann;

$R^7$ für Wasserstoff, $(C_1-C_4)$-Alkyl, Aryl-$(C_1-C_4)$-alkyl-, Hydroxy-$(C_1-C_4)$-alkyl-, Hydroxycarbonyl-$(C_1-C_4)$-alkyl-, ($(C_1-C_4)$-Alkoxycarbonyl)-$(C_1-C_4)$-alkyl-, $R^8R^9N$-CO-$(C_1-C_4)$-alkyl-, $R^{10}$-$SO_2$- oder Aryl steht, wobei $R^7$ dann, wenn diese Gruppe an einem für $R^1R^2N$ stehenden Piperazinorest steht, nicht für carbocydisches Aryl oder carbocydisches Aryl-$(C_1-C_4)$-alkyl- stehen kann;

$R^8$ und $R^9$ für gleiche oder verschiedene Reste aus der Reihe Wasserstoff und $(C_1-C_4)$-Alkyl stehen;

$R^{10}$ für $(C_1-C_4)$-Alkyl, Aryl oder $R^8R^9N$ steht;

Aryl für Phenyl, Naphthyl oder Heteroaryl steht, die alle durch einen oder mehrere gleiche oder verschiedene Substituenten aus der Reihe Halogen, $(C_1-C_4)$-Alkyl, Phenyl, $CF_3$, $NO_2$, OH, -O-$(C_1-C_4)$-Alkyl, -O-$(C_2-C_4)$-Alkyl-O-$(C_1-C_4)$-Alkyl $(C_1-C_2)$-Alkylendioxy, $NH_2$, -NH-$(C_1-C_4)$-Alkyl, -N($(C_1-C_4)$-Alkyl$)_2$, -NH-CHO, -NH-CO-$(C_1-C_4)$-Alkyl, -CN, -CO-$NH_2$, -CO-NH-$(C_1-C_4)$-Alkyl, -CO-N($(C_1-C_4)$-Alkyl$)_2$, -CO-OH, -CO-O-$(C_1-C_4)$-Alkyl, -CHO und -CO-$(C_1-C_4)$-Alkyl substituiert sein können;

Heteroaryl für den Rest eines monocyclischen 5-gliedrigen oder 6-gliedrigen aromatischen Heterocyclus oder eines bicyclischen 8-gliedrigen bis 10-gliedrigen aromatischen Heterocyclus steht, die jeweils ein oder mehrere gleiche oder verschiedene Ringheteroatome aus der Reihe N, O und S enthalten;

m für 0, 1 oder 2 steht;

in allen ihren stereoisomeren Formen und Mischungen davon in allen Verhältnissen, und ihre physiologisch verträglichen Salze,

wobei Verbindungen der Formel I ausgeschlossen sind, in denen gleichzeitig $R^4$ für tert-Butyl oder Trifluormethyl steht, $R^3$ für Phenyl steht, das durch einen oder zwei gleiche oder verschiedene Substituenten aus der Reihe Halogen, OH, -O-$R^{11}$ und $CF_3$ substituiert sein kann, $R^1R^2N$ für $R^{11}$-NH-, $(R^{11})_2N$- oder $R^{12}R^{13}N$-$(CH_2)_p$-NHsteht, p für 2 oder 3 steht, $R^{11}$ für gesättigtes unsubstituiertes $(C_1-C_4)$-Alkyl steht und $R^{12}$ und $R^{13}$ für gleiche oder verschiedene Reste aus der Reihe Wasserstoff und $R^{11}$ stehen oder die Gruppe $R^{12}R^{13}N$ für einen über ein Ringstickstoffatom gebundenen Rest eines 5-gliedrigen oder 6-gliedrigen gesättigten heterocyclischen Ringes steht, der neben dem die Reste $R^{12}$ und $R^{13}$ tragenden Stickstoffatom als weiteres Hetero-Ringglied noch ein Sauerstoffatom, ein Schwefelatom oder ein Stickstoffatom enthalten kann und der durch einen Arylrest oder durch einen Aryl-($C_1$-$C_4$)-alkylrest substituiert sein kann, wobei die Arylgruppe durch einen oder zwei gleiche oder verschiedene Substituenten aus der Reihe Halogen, OH, -O-$R^{11}$ und $CF_3$ substituiert sein kann.

**2.** Verbindungen der Formel I gemäß Anspruch 1, worin
$R^1$ für ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-S(O)$_m$-, $R^5R^6N$ und Aryl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, steht; und
$R^2$ für Wasserstoff, ($C_1$-$C_8$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-S(O)$_m$-; $R^5R^6N$ und Aryl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, steht; oder
$R^1R^2N$ für einen über ein Ringstickstoffatom gebundenen Rest eines 5-gliedrigen, 6-gliedrigen oder 7-gliedrigen gesättigten heterocyclischen Ringes steht, der neben dem die Reste $R^1$ und $R^2$ tragenden Stickstoffatom als weiteres Hetero-Ringglied noch ein Sauerstoffatom, eine Gruppe S(O)$_m$ oder ein einen Rest $R^7$ tragendes Stickstoffatom enthalten kann und der durch einen oder mehrere gleiche oder verschiedene Substituenten aus der Reihe ($C_1$-$C_4$)-Alkyl, Hydroxy, ($C_1$-$C_4$)-Alkoxy, $R^8R^9N$, Hydroxycarbonyl, ($C_1$-$C_4$)-Alkoxycarbonyl und $R^8R^9N$-CO- substituiert sein kann; in allen ihren stereoisomeren Formen und Mischungen davon in allen Verhältnissen, und ihre physiologisch verträglichen Salze.

**3.** Verbindungen der Formel I gemäß Anspruch 1 und/oder 2, worin,
$R^1$ für ($C_1$-$C_4$)-Alkyl, das durch einen oder mehrere gleiche oder verschiedene Substituenten aus der Reihe Hydroxy, ($C_1$-$C_4$)-Alkoxy, ($C_1$-$C_4$)-Alkyl-S(O)$_m$-, $R^5R^6N$ und Aryl substituiert sein kann, oder für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, steht, und $R^2$ für Wasserstoff steht, oder $R^1$ und $R^2$ für gleiches oder verschiedenes ($C_1$-$C_4$)-Alkyl stehen, das durch einen oder mehrere gleiche oder verschiedene Substituenten aus der Reihe Hydroxy, ($C_1$-$C_4$)-Alkoxy, ($C_1$-$C_4$)-Alkyl-S(O)$_m$-, $R^5R^6N$ und Aryl substituiert sein kann;
in allen ihren stereoisomeren Formen und Mischungen davon in allen Verhältnissen, und ihre physiologisch verträglichen Salze.

**4.** Verbindungen der Formel I gemäß einem oder mehreren der Ansprüche 1 bis 3, worin $R^1$ für ($C_3$-$C_9$)-Cycloalkyl, das durch einen oder mehrere gleiche oder verschiedene Substituenten aus der Reihe ($C_1$-$C_4$)-Alkyl, Hydroxy und Amino substituiert sein kann, steht, und $R^2$ für Wasserstoff steht;
in allen ihren stereoisomeren Formen und Mischungen davon in allen Verhältnissen, und ihre physiologisch verträglichen Salze.

**5.** Verbindungen der Formel I gemäß Anspruch 1 undloder 2, worin $R^1R^2N$ für einen unsubstituierten oder substituierten Rest aus der Reihe Piperidino, Morpholino, Thiomorpholino (und dessen S-Oxid und S,S-Dioxid) und Piperazino steht; in allen ihren stereoisomeren Formen und Mischungen davon in allen Verhältnissen, und ihre physiologisch verträglichen Salze.

**6.** Verbindungen der Formel I gemäß einem oder mehreren der Ansprüche 1 bis 5, worin $R^3$ für substituiertes Phenyl steht; in allen ihren stereoisomeren Formen und Mischungen davon in allen Verhältnissen, und ihre physiologisch verträglichen Salze.

**7.** Verbindungen der Formel I gemäß einem oder mehreren der Ansprüche 1 bis 6, worin $R^4$ für ($C_3$-$C_4$)-Alkyl steht; in allen ihren stereoisomeren Formen und Mischungen davon in allen Verhältnissen, und ihre physiologisch verträglichen Salze.

**8.** Verfahren zur Herstellung von Verbindungen der Formel 1 gemäß einem oder mehreren der Ansprüche 1 bis 7, **dadurch gekennzeichnet, daß** ein
4-Hydroxypyrimidin der Formel IV aktiviert wird und dann mit einem Amin der Formel VI umgesetzt wird,

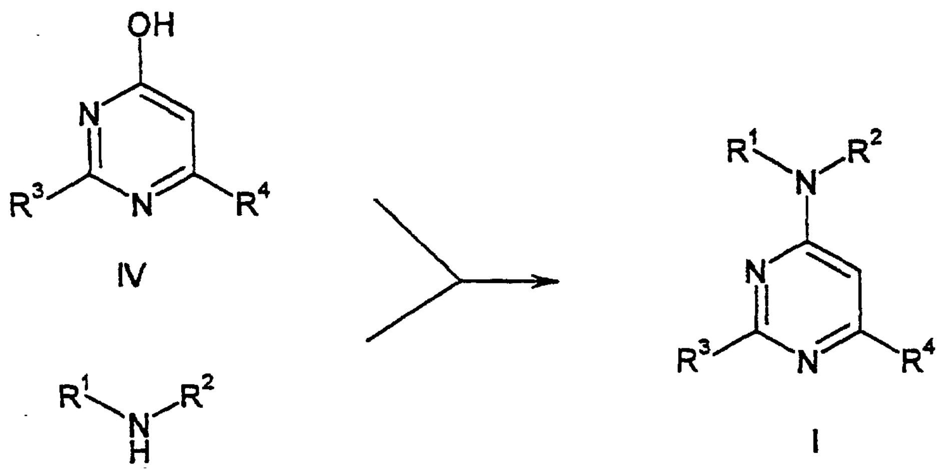

IV

VI

I

wobei $R^1$, $R^2$ $R^3$ und $R^4$ die in den Ansprüchen 1 bis 7 angegebenen Bedeutungen haben.

**9.** Verbindungen der Formel I gemäß einem oder mehreren der Ansprüche 1 bis 7 und/oder ihre physiologisch verträglichen Salze zur Verwendung als Arzneimittel.

**10.** Pharmazeutisches Präparat, **dadurch gekennzeichnet, daß** es eine oder mehrere Verbindungen der Formel I gemäß einem oder mehreren der Ansprüche 1 bis 7 und/oder ihre physiologisch verträglichen Salze und einen pharmazeutisch verträglichen Träger enthält.

**11.** Verbindungen der Formel I gemäß einem oder mehreren der Ansprüche 1 bis 7 und/oder ihre physiologisch verträglichen Salze zur Verwendung als Aktivatoren der löslichen Guanylatcyclase.

**12.** Verbindungen der Formel I gemäß einem oder mehreren der Ansprüche 1 bis 7 und/oder ihre physiologisch verträglichen Salze zur Verwendung in der Therapie oder Prophylaxe von Herz-Kreislauf-Erkrankungen, endothelialer Dysfunktion, diastolischer Dysfunktion, Atherosklerose, Bluthochdruck, Angina pectoris, Thrombosen, Restenosen, Myocardinfarkt, Schlaganfällen, Herzinsuffizienz, Pulmonalhypertonie, erektiler Dysfunktion, Asthma bronchiale, chronischer Niereninsuffizienz, Diabetes oder Leberzirrhose oder zur Verbesserung einer eingeschränkten Lernfähigkeit oder Gedächtnisleistung.

## Claims

**1.** A compound of the formula I,

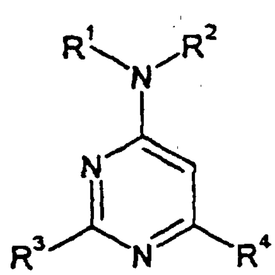

I

in which

$R^1$ is $(C_1-C_8)$-alkyl which can be substituted by one or more identical or . different substituents from the group consisting of hydroxyl, $(C_1-C_4)$-alkoxy, $(C_1-C_4)$-alkyl-$S(O)_m$-, $R^5R^6N$ and aryl, $(C_3-C_9)$-cycloalkyl which can be substituted by one or more identical or different substituents from the group consisting of $(C_1-C_4)$-alkyl, hydroxyl and amino, or the radical of a 5-membered to 7-membered saturated heterocyclic ring which contains one or two identical or different hetero ring members from the group consisting of O, $NR^7$ and $S(O)_m$ and which can be substituted by one or more identical or different substituents from the group consisting of $(C_1-C_4)$-alkyl and aryl-$(C_1-C_4)$-alkyl-; and

$R^2$ is hydrogen, $(C_1-C_8)$-alkyl which can be substituted by one or more identical or different substituents from the group consisting of hydroxyl, $(C_1-C_4)$-alkoxy, $(C_1-C_4)$-alkyl-$S(O)_m$-, $R^5R^6N$ and aryl, $(C_3-C_9)$-cycloalkyl which can be substituted by one or more identical or different substituents from the group consisting of $(C_1-C_4)$-alkyl, hydroxyl and amino, or the radical of a 5-membered to 7-membered saturated heterocyclic ring which contains one or two identical or different hetero ring members from the group consisting of O, $NR^7$ and $S(O)_m$ and which can be substituted by one or more identical or different substituents from the group consisting of $(C_1-C_4)$-alkyl and aryl-$(C_1-C_4)$-alkyl-;

or

$R^1R^2N$ is a radical, bonded via a ring nitrogen atom, of a 5-membered to 7-membered saturated heterocyclic ring which, in addition to the nitrogen atom carrying the radicals $R^1$ and $R^2$, can contain a further hetero ring member from the group consisting of O, $NR^7$ and $S(O)_m$ and which can be substituted by one or more identical or different substituents from the group consisting of $(C_1-C_4)$-alkyl, hydroxyl, $(C_1-C_4)$-alkoxy, $R^8R^9N$, hydroxycarbonyl, $(C_1-C_4)$-alkoxycarbonyl and $R^8R^9N$-CO-;

$R^3$ is phenyl which can be substituted by one or more identical or different substituents from the group consisting of halogen, $(C_1-C_4)$-alkyl, phenyl, $CF_3$, $NO_2$, OH, -O-$(C_1-C_4)$-alkyl, -O-$(C_2-C_4)$-alkyl-O-$(C_1-C_4)$-alkyl, $(C_1-C_2)$-alkylenedioxy, $NH_2$, -NH-$(C_1-C_4)$-alkyl, $N((C_1-C_4)\text{-alkyl})_2$, -NH-CHO, -NH-CO-$(C_1-C_4)$-alkyl, -CN, -CO-$NH_2$, -CO-NH-$(C_1-C_4)$-alkyl, -CO-$N((C_1-C_4)\text{-alkyl})_2$, -CO-OH, -CO-O-$(C_1-C_4)$-alkyl, -CHO and -CO-$(C_1-C_4)$-alkyl;

$R^4$ is $(C_2-C_5)$-alkyl, trifluoromethyl or phenyl which can be substituted by one or more identical or different substituents from the group consisting of halogen, $(C_1-C_4)$-alkyl, phenyl, $CF_3$, $NO_2$, OH, -O-$(C_1-C_4)$-alkyl, -O-$(C_2-C_4)$-alkyl-O-$(C_1-C_4)$-alkyl, $(C_1-C_2)$-alkylenedioxy, $NH_2$, -NH-$(C_1-C_4)$-alkyl, $N((C_1-C_4)\text{-alkyl})_2$, -NH-CHO, -NH-CO-$(C_1-C_4)$-alkyl, -CN, -CO-$NH_2$, -CO-NH-$(C_1-C_4)$-alkyl, -CO-$N((C_1-C_4)\text{-alkyl})_2$, -CO-OH, -CO-O-$(C_1-C_4)$-alkyl, -CHO and -CO-$(C_1-C_4)$-alkyl;

$R^5$ and $R^6$ are identical or different radicals from the group consisting of hydrogen and $(C_1-C_4)$-alkyl or the group $R^5R^6N$ is a radical, bonded via a ring nitrogen atom, of a 5-membered to 7-membered saturated or unsaturated heterocyclic ring which, in addition to the nitrogen atom carrying the radicals $R^5$ and $R^6$, can additionally contain as a further hetero ring member an oxygen atom, a group $S(O)_m$ or a nitrogen atom and which can carry on ring carbon atoms one or more identical or different substituents from the group consisting of $(C_1-C_4)$-alkyl, hydroxyl and amino and can carry on a ring nitrogen atom a radical $R^7$;

$R^7$ is hydrogen, $(C_1-C_4)$-alkyl, aryl-$(C_1-C_4)$-alkyl-, hydroxy-$(C_1-C_4)$-alkyl-, hydroxycarbonyl-$(C_1-C_4)$-alkyl-, $((C_1-C_4)\text{-alkoxycarbonyl})$-$(C_1-C_4)$-alkyl-, $R^8R^9N$-CO-$(C_1-C_4)$-alkyl-, $R^{10}$-$SO_2$- or aryl, where $R^7$, if this group is a piperazino radical representing $R^1R^2N$, cannot be carbocyclic aryl or carbocyclic aryl-$(C_1-C_4)$-alkyl;

$R^8$ and $R^9$ are identical or different radicals from the group consisting of hydrogen and $(C_1-C_4)$-alkyl;

$R^{10}$ is $(C_1-C_4)$-alkyl, aryl or $R^8R^9N$; aryl is phenyl, naphthyl or heteroaryl, which can all be substituted by one or more identical or different substituents from the group consisting of halogen, $(C_1-C_4)$-alkyl, phenyl, $CF_3$, $NO_2$, OH, -O-$(C_1-C_4)$-alkyl, -O-$(C_2-C_4)$-alkyl-O-$(C_1-C_4)$-alkyl, $(C_1-C_2)$-alkylenedioxy, $NH_2$, -NH-$(C_1-C_4)$-alkyl, -$N((C_1-C_4)\text{-alkyl})_2$, -NH-CHO, -NH-CO-$(C_1-C_4)$-alkyl, -CN, -CO-$NH_2$, -CO-NH-$(C_1-C_4)$-alkyl, -CO-$N((C_1-C_4)\text{-alkyl})_2$, -CO-OH, -CO-O-$(C_1-C_4)$-alkyl, -CHO and -CO-$(C_1-C_4)$-alkyl;

heteroaryl is the radical of a monocyclic 5-membered or 6-membered aromatic heterocycle or of a bicyclic 8-membered to 10-membered aromatic heterocycle, each of which contain one or more identical or different ring heteroatoms from the group consisting of N, O and S;

m is 0,1 or 2;

in all its stereoisomeric forms and mixtures thereof in all ratios, or its physiologically tolerable salts, compounds of the formula I being excluded in which simultaneously $R^4$ is tert-butyl or trifluoromethyl, $R^3$ is phenyl which can be substituted by one or two identical or different substituents from the group consisting of halogen, OH, -O-$R^{11}$ and $CF_3$, $R^1R^2N$ is $R^{11}$-NH-, $(R^{11})_2N$- or $R^{12}R^{13}N$-$(CH_2)_p$-NH-, p is 2 or 3, $R^{11}$ is saturated unsubstituted $(C_1-C_4)$-alkyl and $R^{12}$ and $R^{13}$ are identical or different radicals from the group consisting of hydrogen and $R^{11}$ or the group $R^{12}R^{13}N$ is a radical, bonded via a ring nitrogen atom, of a 5-membered or 6-membered saturated heterocyclic ring which, in addition to the nitrogen atom carrying the radicals $R^{12}$ and $R^{13}$, can additionally contain as a further hetero ring member an oxygen atom, a sulfur atom or a nitrogen atom and which can be substituted by an aryl radical or by an aryl-$(C_1-C_4)$-alkyl radical, where the aryl group can be substituted by one or two identical or different substituents from the group consisting of halogen, OH, -O-$R^{11}$ and $CF_3$.

**2.** A compound of the formula I as claimed in claim 1, in which $R^1$ is $(C_1-C_8)$-alkyl which can be substituted by one or more identical or different substituents from the group consisting of hydroxyl, $(C_1-C_4)$-alkoxy, $(C_1-C_4)$-alkyl-$S(O)_m$-, $R^5R^6N$ and aryl, or is $(C_3-C_9)$-cycloalkyl which can be substituted by one or more identical or different substituents from the group consisting of $(C_1-C_4)$-alkyl, hydroxyl and amino; and
$R^2$ is hydrogen, $(C_1-C_8)$-alkyl which can be substituted by one or more identical or different substituents from the group consisting of hydroxyl, $(C_1-C_4)$-alkoxy, $(C_1-C_4)$-alkyl-$S(O)_m$-, $R^5R^6N$ and aryl, or is $(C_3-C_9)$-cycloalkyl which can be substituted by one or more identical or different substituents from the group consisting of $(C_1-C_4)$-alkyl, hydroxyl and amino; or
$R^1R^2N$ is a radical, bonded via a ring nitrogen atom, of a 5-membered, 6-membered or 7-membered saturated heterocyclic ring which, in addition to the nitrogen atom carrying the radicals $R^1$ and $R^2$ can additionally contain as a further hetero ring member an oxygen atom, a group $S(O)_m$ or a nitrogen atom carrying a radical $R^7$ and which can be substituted by one or more identical or different substituents from the group consisting of $(C_1-C_4)$-alkyl, hydroxyl, $(C_1-C_4)$-alkoxy, $R^8R^9N$, hydroxycarbonyl, $(C_1-C_4)$-alkoxycarbonyl and $R^8R^9N$-CO-; in all its stereoisomeric forms and mixtures thereof in all ratios, or its physiologically tolerable salts.

**3.** A compound of the formula I as claimed in claim 1 and/or 2, in which $R^1$ is $(C_1-C_4)$-alkyl which can be substituted by one or more identical or different substituents from the group consisting of hydroxyl, $(C_1-C_4)$-alkoxy, $(C_1-C_4)$-alkyl-$S(O)_m$-, $R^5R^6N$ and aryl, or $(C_3-C_9)$-cycloalkyl which can be substituted by one or more identical or different substituents from the group consisting of $(C_1-C_4)$-alkyl, hydroxyl and amino, and $R^2$ is hydrogen, or $R^1$ and $R^2$ are identical or different $(C_1-C_4)$-alkyl which can be substituted by one or more identical or different substituents from the group consisting of hydroxyl, $(C_1-C_4)$-alkoxy, $(C_1-C_4)$-alkyl-$S(O)_m$-, $R^5R^6N$ and aryl;
in all its stereoisomeric forms and mixtures thereof in all ratios, or its physiologically tolerable salts.

**4.** A compound of the formula I as claimed in one or more of claims 1 to 3, in which $R^1$ is $(C_3-C_9)$-cycloalkyl which can be substituted by one or more identical or different substituents from the group consisting of $(C_1-C_4)$-alkyl, hydroxyl and amino, and $R^2$ is hydrogen;
in all its stereoisomeric forms and mixtures thereof in all ratios, or its physiologically tolerable salts.

**5.** A compound of the formula I as claimed in claim 1 and/or 2, in which $R^1R^2N$ is an unsubstituted or substituted radical from the group consisting of piperidino, morpholino, thiomorpholino (and its S-oxide and S,S-dioxide) and piperazino; in all its stereoisomeric forms and mixtures thereof in all ratios, or its physiologically tolerable salts.

**6.** A compound of the formula I as claimed in one or more of claims 1 to 5, in which $R^3$ is substituted phenyl; in all its stereoisomeric forms and mixtures thereof in all ratios, or its physiologically tolerable salts.

**7.** A compound of the formula I as claimed in one or more of claims 1 to 6, in which $R^4$ is $(C_3-C_4)$-alkyl; in all its stereoisomeric forms and mixtures thereof in all ratios, or its physiologically tolerable salts.

**8.** A process for the preparation of compounds of the formula I as claimed in one or more of claims 1 to 7, which comprises activating a 4-hydroxypyrimidine of the formula IV and then reacting it with an amine of the formula VI,

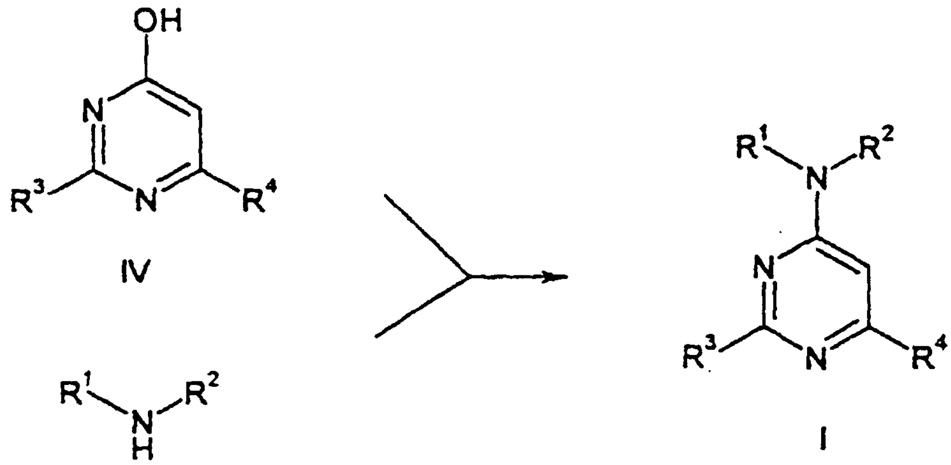

IV

VI

I

where $R^1$, $R^2$, $R^3$ and $R^4$ have the meanings indicated in claims 1 to 7.

**9.** A compound of the formula I as claimed in one or more of claims 1 to 7 and/or its physiologically tolerable salts for use as a pharmaceutical.

**10.** A pharmaceutical preparation, which contains one or more compounds of the formula I as claimed in one or more of claims 1 to 7 and/or its/their physiologically tolerable salts and a pharmaceutically tolerable carrier.

**11.** A compound of the formula I as claimed in one or more of claims 1 to 7 and/or its physiologically tolerable salts for use as activators of soluble guanylate cyclase.

**12.** A compound of the formula I as claimed in one or more of claims 1 to 7 and/or its physiologically tolerable salts for use in the therapy or prophylaxis of cardiovascular disorders, endothelial dysfunction, diastolic dysfunction, atherosclerosis, high blood pressure, angina pectoris, thromboses, restenoses, myocardial infarct, strokes, cardiac insufficiency, pulmonary hypertension, erectile dysfunction, bronchial asthma, chronic renal insufficiency, diabetes or liver cirrhosis or for improving restricted learning capacity or memory power.

**Revendications**

**1.** Composés de formule I

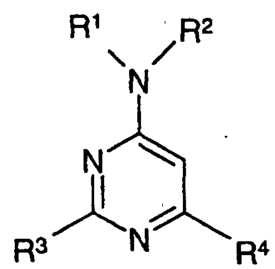

I

où
$R^1$ représente alkyle en $C_1$-$C_8$ qui peut être substitué par un où plusieurs substituants identiques ou différents de la série de hydroxyle, alcoxy en $C_1$-$C_4$, alkyle en $C_1$-$C_4$-$S(O)_m$-, $R^5R^6N$ et aryle, cycloalkyle en $C_3$-$C_9$ qui peut être substitué par un ou plusieurs substituants identiques ou différents de la série de alkyle en $C_1$-$C_4$, hydroxyle et

amino, ou le reste d'un cycle hétérocyclique saturé à 5 à 7 chaînons qui contient 1 ou 2 chaînons de l'hétérocycle identiques ou différents de la série de O, $NR^7$ et $S(O)_m$ et qui peut être substitué par un ou plusieurs substituants identiques ou différents de la série de alkyle en $C_1$-$C_4$ et aryl-alkyle en $C_1$-$C_4$ ;

et

$R^2$ représente l'hydrogène, alkyle en $C_1$-$C_8$ qui peut être substitué par un ou plusieurs substituants identiques ou différents de la série de hydroxyle, alcoxy en $C_1$-$C_4$, alkyle en $C_1$-$C_4$-$S(O)_m$-, $R^5R^6N$ et aryle, cycloalkyle en $C_3$-$C_9$ qui peut être substitué par un ou plusieurs substituants identiques ou différents de la série de alkyle en $C_1$-$C_4$, hydroxyle et amino, ou le reste d'un cycle hétérocyclique saturé à 5 à 7 chaînons qui contient 1 ou 2 chaînons de l'hétérocycle identiques ou différents de la série de O, $NR^7$ et $S(O)_m$ et qui peut être substitué par un ou plusieurs substituants identiques ou différents de la série de alkyle en $C_1$-$C_4$ et aryl-alkyle en $C_1$-$C_4$;

ou

$R^1R^2N$ représente un reste d'un cycle hétérocyclique saturé à 5 à 7 chaînons lié par le biais d'un atome d'azote du cycle, qui, outre l'atome d'azote portant les restes $R^1$ et $R^2$, peut contenir un autre chaînon de l'hétérocycle de la série de O, $NR^7$ et $S(O)_m$ et qui peut être substitué par un ou plusieurs substituants identiques ou différents de la série de alkyle en $C_1$-$C_4$, hydroxyle, alcoxy en $C_1$-$C_4$, $R^8R^9N$, hydroxycarbonyle, alcoxy en $C_1$-$C_4$-carbonyle et $R^8R^9N$-CO-;

$R^3$ représente phényle qui peut être substitué par un ou plusieurs substituants identiques ou différents de la série de halogène, alkyle en $C_1$-$C_4$, phényle, $CF_3$, $NO_2$, OH, -O-alkyle en $C_1$-$C_4$, -O-alkyle en $C_2$-$C_4$-O-alkyle en $C_1$-$C_4$, alkylène en $C_1$-$C_2$-dioxy, $NH_2$, -NH-alkyle en $C_1$-$C_4$, -N(alkyle en $C_1$-$C_4)_2$, -NH-CHO, -NH-CO-alkyle en $C_1$-$C_4$, -CN, -CO-$NH_2$, -CO-NH-alkyle en $C_1$-$C_4$, -CO-N(alkyle en $C_1$-$C_4)_2$, -CO-OH, -CO-O-alkyle en $C_1$-$C_4$, -CHO et -CO-alkyle en $C_1$-$C_4$ ;

$R^4$ représente alkyle en $C_2$-$C_5$, trifluorométhyle ou phényle qui peut être substitué par un ou plusieurs substituants identiques ou différents de la série de halogène, alkyle en $C_1$-$C_4$, phényle, $CF_3$, $NO_2$, OH, -O-alkyle en $C_1$-$C_4$, -O-alkyle en $C_2$-$C_4$-O-alkyle en $C_1$-$C_4$, alkylène en $C_1$-$C_2$-dioxy, $NH_2$, -NH-alkyle en $C_1$-$C_4$, -N(alkyle en $C_1$-$C_4)_2$, -NH-CHO, -NH-CO-alkyle en $C_1$-$C_4$, -CN, -CO-$NH_2$, -CO-NH-alkyle en $C_1$-$C_4$, -CO-N(alkyle en $C_1$-$C_4)_2$, -CO-OH, -CO-O-alkyle en $C_1$-$C_4$, -CHO et -CO-alkyle en $C_1$-$C_4$ ;

$R^5$ et $R^6$ représentent des restes identiques ou différents de la série de hydrogène et alkyle en $C_1$-$C_4$ ou le groupe $R^5R^6N$ représente un reste lié par le biais d'un atome d'azote du cycle d'un cycle hétérocyclique saturé ou insaturé à 5 à 7 chaînons qui, outre l'atome d'azote portant les restes $R^5$ et $R^6$, peut contenir encore, comme autre chaînon de l'hétérocycle, un atome d'oxygène, un groupe $S(O)_m$ ou un atome d'azote et qui peut porter sur des atomes de carbone du cycle un ou plusieurs substituants identiques ou différents de la série de alkyle en $C_1$-$C_4$, hydroxyle et amino et qui peut porter un reste $R^7$ au niveau d'un atome d'azote du cycle ;

$R^7$ représente l'hydrogène, alkyle en $C_1$-$C_4$, aryl-alkyle en $C_1$-$C_4$, hydroxyalkyle en $C_1$-$C_4$, hydroxycarbonyl-alkyle en $C_1$-$C_4$, (alcoxy en $C_1$-$C_4$-carbonyl)-alkyle en $C_1$-$C_4$, $R^8R^9N$-CO-(alkyle en $C_1$-$C_4$), $R^{10}$-$SO_2$- ou aryle, où $R^7$, quand ce groupe est situé au niveau d'un reste pipérazino représentant $R^1R^2N$, ne peut pas représenter aryle carbocyclique ou aryl-alkyle en $C_1$-$C_4$ carbocyclique,

$R^8$ et $R^9$ représentent des restes identiques ou différents de la série de l'hydrogène et alkyle en $C_1$-$C_4$;

$R^{10}$ représente alkyle en $C_1$-$C_4$, aryle ou $R^8R^9N$;

aryle représente phényle, naphtyle ou hétéroaryle qui peuvent tous être substitués par un ou plusieurs substituants identiques ou différents de la série de halogène, alkyle en $C_1$-$C_4$, phényle, $CF_3$, $NO_2$, OH, -O-alkyle en $C_1$-$C_4$, -O-alkyle en $C_2$-$C_4$-O-alkyle en $C_1$-$C_4$, alkylène en $C_1$-$C_2$-dioxy, $NH_2$, -NH-alkyle en $C_1$-$C_4$, -N(alkyle en $C_1$-$C_4)_2$, -NH-CHO, -NH-CO-alkyle en $C_1$-$C_4$, -CN, -CO-$NH_2$, -CO-NH-alkyle en $C_1$-$C_4$, -CO-N(alkyle en $C_1$-$C_4)_2$, -CO-OH, -CO-O-alkyle en $C_1$-$C_4$, -CHO et -CO-alkyle en $C_1$-$C_4$ ;

hétéroaryle représente le reste d'un hétérocycle aromatique à 5 ou 6 chaînons monocyclique ou d'un hétérocycle aromatique à 8 à 10 chaînons bicyclique qui peuvent contenir chacun un ou plusieurs hétéroatomes du cycle identiques ou différents de la série de N, O et S ;

m représente 0, 1 ou 2 ;

dans toutes leurs formes stéréoisomères et les mélanges de celles-ci en toutes proportions, et leurs sels physiologiquement acceptables,

où sont exclus les composés de formule I où simultanément $R^4$ représente tert-butyle ou trifluorométhyle, $R^3$ représente phényle qui peut être substitué par un ou deux substituants identiques ou différents de la série de halogène, OH, -O-$R^{11}$ et $CF_3$, $R^1R^2N$ représente $R^{11}$-NH-, $(R^{11})_2N$- ou $R^{12}R^{13}N$-$(CH_2)_p$-NH-, p représente 2 ou 3, $R^{11}$ représente alkyle en $C_1$-$C_4$ non substitué saturé et $R^{12}$ et $R^{13}$ représentent des restes identiques ou différents de la série de l'hydrogène et $R^{11}$, ou le groupe $R^{12}R^{13}N$ représente un reste d'un cycle hétérocyclique saturé à 5 ou 6 chaînons lié par le biais d'un atome d'azote du cycle, qui, outre l'atome d'azote portant les restes $R^{12}$ et $R^{13}$, peut contenir encore comme autre chaînon de l'hétérocycle un atome d'oxygène, un atome de soufre ou un atome d'azote et qui peut être substitué par un reste aryle ou par un reste aryl-alkyle en $C_1$-$C_4$, où le groupe aryle peut être substitué par un ou deux substituants identiques ou différents de la série de halogène, OH, -O-$R^{11}$ et $CF_3$.

**2.** Composés de formule I selon la revendication 1, où
$R^1$ représente alkyle en $C_1$-$C_8$ qui peut être substitué par un ou plusieurs substituants identiques ou différents de la série de hydroxyle, alcoxy en $C_1$-$C_4$, alkyle en $C_1$-$C_4$-$S(O)_m$-, $R^5R^6N$ et aryle, ou cycloakyle en $C_3$-$C_9$ qui peut être substitué par un ou plusieurs substituants identiques ou différents de la série de alkyle en $C_1$-$C_4$, hydroxyle et amino ; et
$R^2$ représente l'hydrogène, alkyle en $C_1$-$C_8$, qui peut être substitué par un ou plusieurs substituants identiques ou différents de la série de hydroxyle, alcoxy en $C_1$-$C_4$, alkyle en $C_1$-$C_4$-$S(O)_m$-, $R^5R^6N$ et aryle, ou cycloalkyle en $C_3$-$C_9$ qui peut être substitué par un ou plusieurs substituants identiques ou différents de la série de alkyle en $C_1$-$C_4$, hydroxyle et amino ; ou
$R^1R^2N$ représente un reste d'un cycle hétérocyclique saturé à 5, 6 ou 7 chaînons lié par le biais d'un atome d'azote du cycle, qui, outre l'atome d'azote portant les restes $R^1$ et $R^2$, peut contenir encore comme autre chaînon de l'hétérocycle un atome d'oxygène, un groupe $S(O)_m$ ou un atome d'azote portant un reste $R^7$ et qui peut être substitué par un ou plusieurs substituants identiques ou différents de la série de alkyle en $C_1$-$C_4$, hydroxyle, alcoxy en $C_1$-$C_4$, $R^8R^9N$, hydroxycarbonyle, alcoxy en $C_1$-$C_4$-carbonyle et $R^8R^9N$-CO- ; dans toutes leurs formes stéréoisomères et les mélanges de celles-ci en toutes proportions, et leurs sels physiologiquement acceptables.

**3.** Composés deformule I selon la revendication 1 et/ou 2, où
$R^1$ représente alkyle en $C_1$-$C_4$ qui peut être substitué par un ou plusieurs substituants identiques ou différents de la série de hydroxyle, alcoxy en $C_1$-$C_4$, alkyle en $C_1$-$C_4$-$S(O)_m$-, $R^5R^6N$ et aryle, ou cycloalkyle en $C_3$-$C_9$ qui peut être substitué par un ou plusieurs substituants identiques ou différents de la série de alkyle en $C_1$-$C_4$, hydroxyle et amino, et $R^2$ représente l'hydrogène, ou bien $R^1$ et $R^2$ représentent alkyle en $C_1$-$C_4$ identique ou différent qui peut être substitué par un ou plusieurs substituants identiques ou différents de la série de hydroxyle, alcoxy en $C_1$-$C_4$, alkyle en $C_1$-$C_4$-$S(O)_m$-, $R^5R^6N$ et aryle ;
dans toutes leurs formes stéréoisomères et les mélanges de celles-ci en toute proportions, et leurs sels physiologiquement acceptables.

**4.** Composés de formule I selon une ou plusieurs des revendications 1 à 3, où $R^1$ représente cycloalkyle en $C_3$-$C_9$ qui peut être substitué par un ou plusieurs substituants identiques ou différents de la série de alkyle en $C_1$-$C_4$, hydroxyle et amino, et $R^2$ représente l'hydrogène ;
dans toutes leurs forme stéréoisomères et les mélanges de celles-ci en toutes proportions, et leurs sels physiologiquement acceptables.

**5.** Composés de formule I selon la revendication 1 et/ou 2, où $R^1R^2N$ représente un reste non substitué ou substitué de la série de pipéridino, morpholino, thiomorpholino (et son S-oxyde et S,S-dioxyde) et pipérazino ; dans toutes leurs formes stéréoisomères et les mélanges de celles-ci en toutes proportions, et leurs sels physiologiquement acceptables.

**6.** Composés de formule I selon une ou plusieurs des revendications 1 à 5, où $R^3$ représente phényle substitué ; dans toutes leurs formes stéréoisomères et les mélanges de celles-ci en toutes proportions, et leurs sels physiologiquement acceptables.

**7.** Composés de formule I selon une ou plusieurs des revendications 1 à 6, où $R^4$ représente alkyle en $C_3$-$C_4$ ; dans toutes leurs formes stéréoisomères et les mélanges de celles-ci en toutes proportions, et leurs sels physiologiquement acceptables.

**8.** Procédé de préparation de composés de formule I selon une
ou plusieurs des revendications 1 à 7, **caractérisé en ce qu'**une 4-hydroxypyrimidine de formule IV est activée puis mise à réagir avec une amine de formule VI

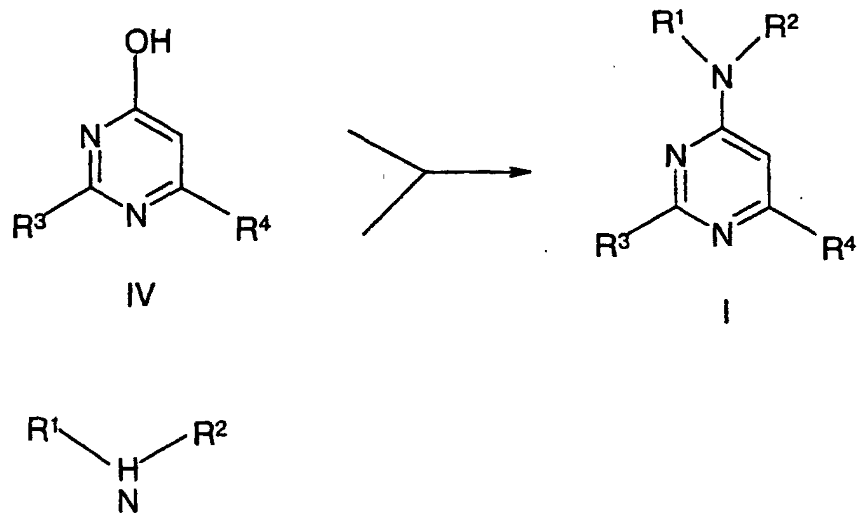

IV

I

VI

où $R^1$, $R^2$, $R^3$ et $R^4$ ont les significations indiquées dans les revendications 1 à 7.

**9.** Composés de formule I selon une ou plusieurs des revendications 1 à 7 et/ou leurs sels physiologiquement acceptables destinés à être utilisés comme médicaments.

**10.** Préparation pharmaceutique, **caractérisée en ce qu'**elle contient un ou plusieurs composés de formule I selon une ou plusieurs des revendications 1 à 7 et/ou leurs sels physiologiquement acceptables et un support pharmaceutiquement acceptable.

**11.** Composés de formule I selon une ou plusieurs des revendications 1 à 7 et/ou leurs sels physiologiquement acceptables destinés à être utilisés comme activateurs de la guanylate cyclase soluble.

**12.** Composés de formule I selon une ou plusieurs des revendications 1 à 7 et/ou leurs sels physiologiquement acceptables destinés à être utilisés dans thérapie ou la prophylaxie des maladies cardiovasculaires, du dysfonctionnement endothélial, du dysfonctionnement diastolique, de l'athérosclérose, de l'hypertension sanguine, de l'angine de poitrine, des thromboses, des resténoses, de l'infarctus du myocarde, des apoplexies cérébrales, de l'insuffisance cardiaque, de l'hypertonie pulmonaire, du dysfonctionnement érectile, de l'asthme bronchique, de l'insuffisance rénale chronique, du diabète ou de la cirrhose du foie ou pour améliorer une capacité d'apprentissage ou des performances de mémoire limitées.